(19) Europäisches Patentamt
European Patent Office
Office européen des brevets

(11) EP 1 368 032 B1

(12) EUROPEAN PATENT SPECIFICATION

(45) Date of publication and mention of the grant of the patent:
**11.06.2008 Bulletin 2008/24**

(21) Application number: **01274491.8**

(22) Date of filing: **19.12.2001**

(51) Int Cl.:
***A61K 31/472*** (2006.01) ***A61K 31/4725*** (2006.01)
***C07D 217/26*** (2006.01) ***C07D 401/12*** (2006.01)
***C07D 409/14*** (2006.01) ***C07D 401/14*** (2006.01)

(86) International application number:
**PCT/US2001/045857**

(87) International publication number:
**WO 2003/024453 (27.03.2003 Gazette 2003/13)**

(54) **EXCITATORY AMINO ACID RECEPTOR ANTAGONISTS**

EXZITATORISCHE AMINOSÄURE-REZEPTOR-ANTAGONISTEN

ANTAGONISTES DES RECEPTEURS D'AMINOACIDES EXCITATOIRES

(84) Designated Contracting States:
**AT BE CH CY DE DK ES FI FR GB GR IE IT LI LU MC NL PT SE TR**

(30) Priority: **05.01.2001 EP 01500004**

(43) Date of publication of application:
**10.12.2003 Bulletin 2003/50**

(60) Divisional application:
**08153665.8**

(73) Proprietor: **ELI LILLY AND COMPANY**
**Indianapolis, Indiana 46285 (US)**

(72) Inventors:
- **BLEISCH, Thomas, John**
  **Noblesville, IN 46060 (US)**
- **MARTINEZ PEREZ, Jose Antonio**
  **Lilly S. A.**
  **E-28100 Alcobendas (ES)**
- **ORNSTEIN, Paul, Leslie**
  **Carmel, IN 46032 (US)**
- **MAY, Scott, Allan**
  **Noblesville, IN 46060 (US)**
- **RATZ, Andrew, Michael**
  **Zionsville, IN 46077 (US)**
- **WILSON, Thomas, Michael**
  **Speedway, IN 46224 (US)**

(74) Representative: **Suarez-Miles, Ana Sanchiz et al**
**Eli Lilly and Company Limited**
**Lilly Research Centre**
**Erl Wood Manor**
**Sunninghill Road**
**Windlesham, Surrey GU20 6PH (GB)**

(56) References cited:
**EP-A- 0 590 789**
**WO-A-01/46173**
**US-A- 5 446 051**
**WO-A-01/01972**
**WO-A-98/45270**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 368 032 B1

## Description

## BACKGROUND OF THE INVENTION

In the mammalian central nervous system (CNS), the transmission of nerve impulses is controlled by the interaction between a neurotransmitter, that is released by a sending neuron, and a surface receptor on a receiving neuron, which causes excitation of this receiving neuron. L-Glutamate, which is the most abundant neurotransmitter in the CNS, mediates the major excitatory pathways in mammals, and is referred to as an excitatory amino acid (EAA). The receptors that respond to glutamate are called excitatory amino acid receptors (EAA receptors). See Watkins & Evans, Ann. Rev. Pharmacol. Toxicol., 21, 165 (1981); Monaghan, Bridges, and Cotman, Ann. Rev. Pharmacol. Toxicol., 29. 365 (1989); Watkins, Krogsgaard-Larsen, and Honore, Trans. Pharm. Sci., 11, 25 (1990). The excitatory amino acids are of great physiological importance, playing a role in a variety of physiological processes, such as long-term potentiation (learning and memory), the development of synaptic plasticity, motor control, respiration, cardiovascular regulation, and sensory perception.

Excitatory amino acid receptors are classified into two general types. Receptors that are directly coupled to the opening of cation channels in the cell membrane of the neurons are termed "ionotropic." This type of receptor has been subdivided into at least three subtypes, which are defined by the depolarizing actions of the selective agonists N-methyl-D-aspartate (NMDA), $\alpha$-amino-3-hydroxy-5-methylisoxazole-4-propionic acid (AMPA), and kainic acid (KA). Molecular biological studies have established that AMPA receptors are composed of subunits ($GluR_1$ - $GluR_4$), which can assemble to form functional ion channels. Five kainate receptors have been identified which are classified as either High Affinity (KA1 and KA2) or Low Affinity (composed of $GluR_5$, $GluR_6$, and/or $GluR_7$ subunits). Bleakman et al., Molecular Pharmacology, 49, No.4, 581,(1996). The second general type of receptor is the G-protein coupled or second messenger-linked "metabotropic" excitatory amino acid receptor. This second type is coupled to multiple second messenger systems that lead to enhanced phosphoinositide hydrolysis, activation of phospholipase D, increases or decreases in cAMP formation, and changes in ion channel function. Schoepp and Conn, Trends in Pharmacol. Sci., 14, 13 (1993). Both types of excitatory amino acid receptor appear not only to mediate normal synaptic transmission along excitatory pathways, but also to participate in the modification of synaptic connections during development and throughout life. Schoepp, Bockaert, and Sladeczek, Trends in Pharmacol. Sci., 11, 508 (1990); McDonald and Johnson, Brain Research Reviews, 15, 41 (1990).

The excessive or inappropriate stimulation of excitatory amino acid receptors leads to neuronal cell damage or loss by way of a mechanism known as excitotoxicity. This process has been suggested to mediate neuronal degeneration in a variety of neurological disorders and conditions. The medical consequences of such neuronal degeneration makes the abatement of these degenerative neurological processes an important therapeutic goal. For instance, excitatory amino acid receptor excitotoxicity has been implicated in the pathophysiology of numerous neurological disorders, including the etiology of cerebral deficits subsequent to cardiac bypass surgery and grafting, stroke, cerebral ischemia, spinal cord lesions resulting from trauma or inflammation, perinatal hypoxia, cardiac arrest, and hypoglycemic neuronal damage. In addition, excitotoxicity has been implicated in chronic neurodegenerative conditions including Alzheimer's Disease, Huntington's Chorea, inherited ataxias, AIDS-induced dementia, amyotrophic lateral sclerosis, idiopathic and drug-induced Parkinson's Disease, as well as ocular damage and retinopathy. Other neurological disorders implicated with excitotoxicity and/or glutamate dysfunction include muscular spasticity including tremors, drug tolerance and withdrawal, brain edema, convulsive disorders including epilepsy, depression, anxiety and anxiety related disorders such as post-traumatic stress syndrome, tardive dyskinesia, and psychosis related to depression, schizophrenia, bipolar disorder, mania, and drug intoxication or addiction (see generally United States Patent No. 5,446,051 and 5,670,516). Excitatory amino acid receptor antagonists may also be useful as analgesic agents and for treating or preventing various forms of headache, including cluster headache, tension-type headache, and chronic daily headache. In addition, published International Patent application WO 98/45720 reports that excitatory amino acid receptor excitotoxicity participates in the etiology of acute and chronic pain states including severe pain, intractable pain, neuropathic pain, post-traumatic pain.

It is also known that trigeminal ganglia, and their associated nerve pathways, are associated with painful sensations of the head and face such as headache and, in particular, migraine. Moskowitz (Cephalalgia, 12, 5-7, (1992) proposed that unknown triggers stimulate the trigeminal ganglia which in turn innervate vasculature within cephalic tissue, giving rise to the release of vasoactive neuropeptides from axons innervating the vasculature. These neuropeptides initiate a series of events leading to neurogenic inflammation of the meninges, a consequence of which is pain. This neurogenic inflammation is blocked by sumatriptan at doses similar to those required to treat acute migraine in humans. However, such doses of sumatriptan are associated with contraindications as a result of sumatriptan's attendant vasoconstrictive properties.(see MacIntyre, P.D., et al., British Journal of Clinical Pharmacology, 34, 541-546 (1992); Chester, A.H., et al., Cardiovascular Research, 24, 932-937 (1990); Conner, H.E., et al., European Journal of Pharmacology, 161, 91-94 (1990)). Recently, it has been reported that all five members of the kainate subtype of ionotropic

glutamate receptors are expressed on rat trigeminal ganglion neurons, and in particular, high levels of $GluR_5$ and KA2 have been observed. (Sahara *et al., The Journal of Neuroscience*, 17(17), 6611 (1997)). As such, migraine presents yet another neurological disorder which may be implicated with glutamate receptor excitotoxicity.

The use of a neuroprotective agent, such as an excitatory amino acid receptor antagonist, is believed to be useful in treating or preventing all of the aforementioned disorders and/or reducing the amount of neurological damage associated with these disorders. For example, studies have shown that AMPA receptor antagonists are neuroprotective in focal and global ischemia models. The competitive AMPA receptor antagonist NBQX (2,3-dihydroxy-6-nitro-7-sulfamoylbenzo[*f*]quinoxaline) has been reported effective in preventing global and focal ischemic damage. Sheardown et al., Science, 247, 571 (1900); Buchan et al., Neuroreport, 2, 473 (1991); LePeillet et al., Brain Research, 571, 115 (1992). The noncompetitive AMPA receptor antagonists GKYI 52466 has been shown to be an effective neuroprotective agent in rat global ischemia models. LaPeillet et al., Brain Research, 571, 115 (1992). European Patent Application Publication No. 590789A1 and United States Patents No. 5,446,051 and 5,670,516 disclose that certain decahydroisoquinoline derivative compounds are AMPA receptor antagonists and as such, are useful in the treatment of a multitude of disorders conditions, including pain and migraine headache. WO 98/45270 discloses that certain decahydroisoquinoline derivative compounds are selective antagonists of the $iGluR_5$ receptor and are useful for the treatment of various types of pain, including; severe, chronic, intractable, and neuropathic pain.

In accordance with the present invention. Applicants have discovered novel compounds that are antagonists of the $iGluR_5$ receptor subtype and, thus, could be useful in treating the multitude of neurological disorders or neurodegenerative diseases, as discussed above. Such antagonists could address a long felt need for safe and effective treatments for neruological disorders, without attending side effects. The treatment of neurological disorders and neurodegenerative diseases is hereby furthered.

## SUMMARY OF THE INVENTION

The present invention provides a compound of Formula I

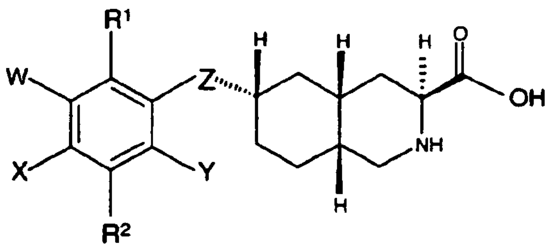

Formula I

wherein

Z represents an oxygen atom;
$R^1$ represents tetrazole;
$R^2$ represents hydrogen;
W represents halo; and
X, and Y each independently represent hydrogen;
or a pharmaceutically acceptable salt thereof.

In another embodiment, the present invention provides the use of a compound of Formula I or a pharmaceutically acceptable salt thereof for the manufacture of a medicament for treating or preventing a neurological disorder, or neurodegenerative condition. Examples of such neurological disorders, or neurodegenerative conditions, include: cerebral deficits subsequent to cardiac bypass surgery and grafting; stroke; cerebral ischemia; spinal cord lesions resulting from trauma or inflammation; perinatal hypoxia; cardiac arrest; hypoglycemic neuronal damage; Alzheimer's Disease; Huntington's Chorea; inherited ataxias; AIDS-induced dementia; amyotrophic lateral sclerosis; idiopathic and drug-induced Parkinson's Disease; ocular damage and retinopathy; muscular spasticity including tremors; drug tolerance and withdrawal; brain edema; convulsive disorders including epilepsy; depression; anxiety and anxiety related disorders such as post-traumatic stress syndrome; tardive dyskinesia; psychosis related to depression, schizophrenia, bipolar disorder, mania, and drug intoxication or addiction; headache, including cluster headache, tension-type headache, and chronic

daily headache; migraine; and acute and chronic pain states including severe pain, intractable pain, neuropathic pain, and post-traumatic pain.

More specifically, the present invention provides the use of a compound of Formula I or a pharmaceutically acceptable salt thereof for the manufacture of a medicament for treating or preventing pain or migraine.

In addition, the present invention provides pharmaceutical compositions of compounds of Formula I, including the pharmaceutically acceptable salts thereof, comprising, a compound of Formula I in combination with a pharmaceutically acceptable carrier, diluent or excipient. This invention also encompasses novel intermediates, and processes for the synthesis of the compounds of Formula I.

Specifically, the present invention also provides a process for making compounds of the Formula:

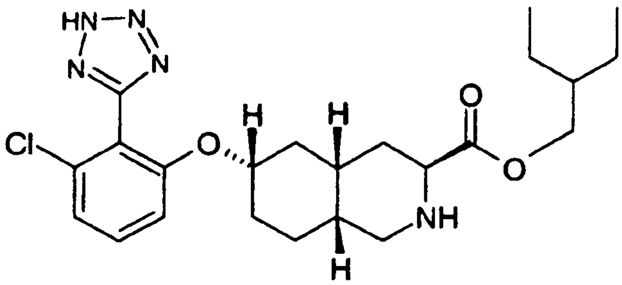

comprising combining a compound of the structure:

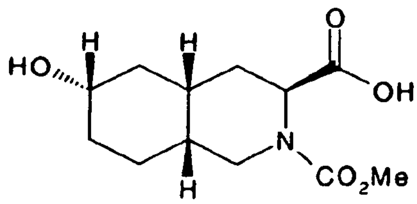

with a suitable base in a suitable solvent, followed by addition of a compound of the structure:

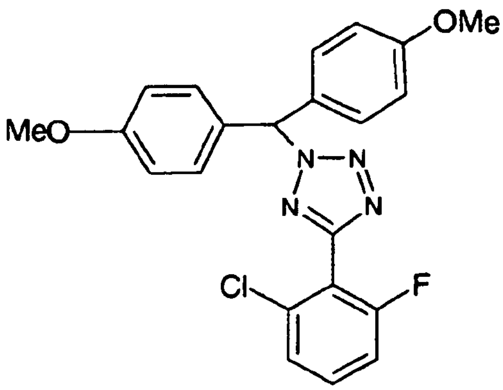

followed by esterification to a compound of the structure:

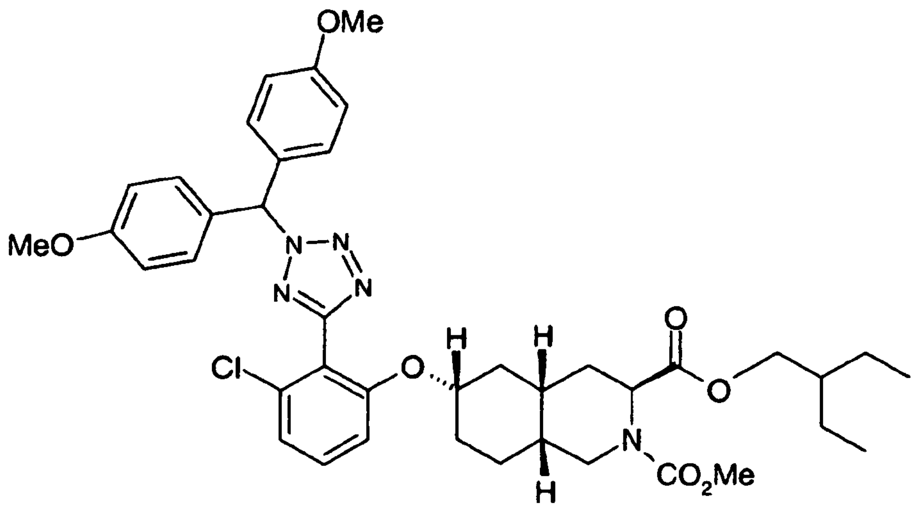

followed by removal of the nitrogen protecting groups, and precipitation with a suitable acid.

In a further embodiment, the present invention provides yet another process for synthesizing a compound of the Formula:

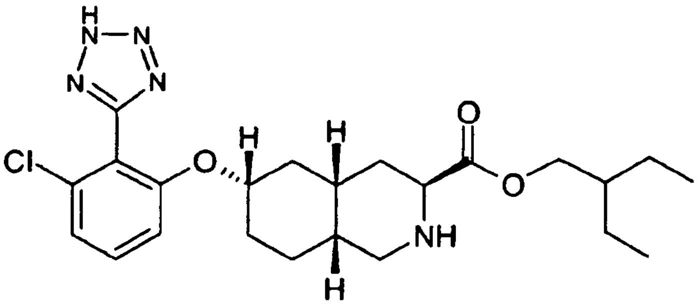

comprising combining a compound of the structure:

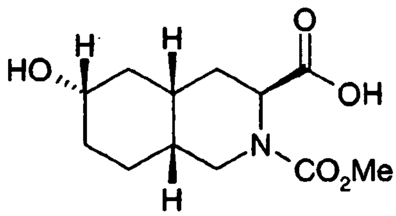

with a suitable base in a suitable solvent, followed by addition of a compound of the structure:

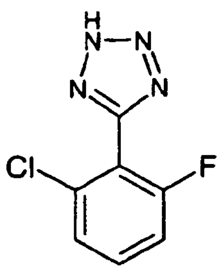

followed by deprotection of the nitrogen group, precipitation with a suitable acid, and crystallization of the hydrate salt of the structure:

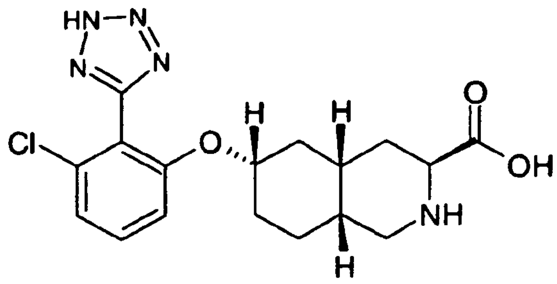

followed by treatment with a suitable alcohol in the presence of a suitable acid to effect the one step esterification and crystallization of the compound of the Formula:

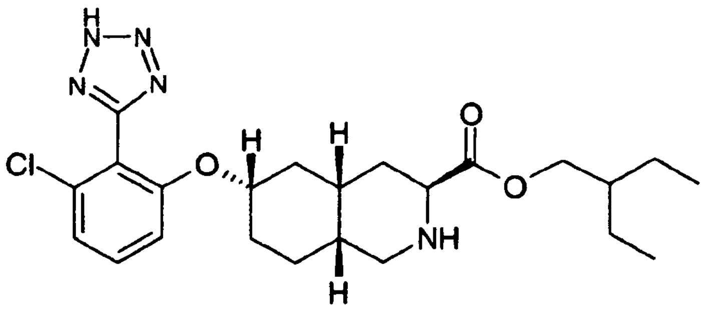

The present invention also provides the use of a compound of Formula I or Formula I(a) for the manufacture of a medicament for treating or preventing a neurological disorder, or neurodegenerative condition.

More specifically, the present invention provides the use of a compound of Formula I or Formula I(a) for the manufacture of a medicament for treating or preventing pain or migraine.

## DETAILED DESCRIPTION OF THE INVENTION

The present invention provides compounds functional as $iGluR_5$ receptor antagonists as well as pharmaceutically acceptable salts, prodrugs, and compositions thereof. These compounds are useful in treating or preventing neurological disorders, or neurodegenerative diseases, particularly pain and migraine.

In addition, it should be understood by the skilled artisan that all of the compounds useful for the methods of the present invention are available for prodrug formulation. As used herein, the term "prodrug" refers to a compound of Formula Ia below:

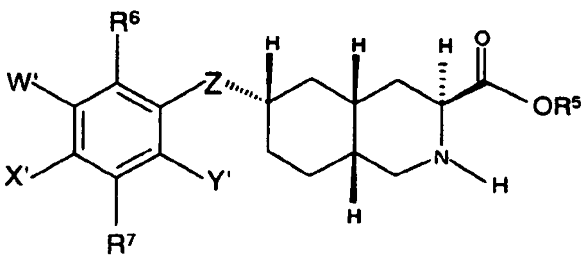

Formula Ia

wherein

Z represents an oxygen atom;
$R^5$ represents ($C_1$-$C_6$) alkyl;
$R^6$ represents tetrazole;
$R^7$ represents hydrogen;
W' represents halo; and
X' and Y' each independently represent hydrogen;
or a pharmaceutically acceptable salt thereof.

It is understood that the $iGluR_5$ receptor antagonists of the present invention may exist as pharmaceutically acceptable salts and, as such, salts are therefore included within the scope of the present invention. The term "pharmaceutically acceptable salt" as used herein, refers to salts of the compounds provided by, or employed in the present invention which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts.

It will be understood by the skilled reader that most or all of the compounds used in the present invention are capable of forming salts, and that the salt forms of pharmaceuticals are commonly used, often because they are more readily crystallized and purified than are the free bases. In all cases, the use of the pharmaceuticals described herein as salts is contemplated in the description herein, and often is preferred, and the pharmaceutically acceptable salts of all of the compounds are included in the names of them.

Acids commonly employed to form acid addition salts are inorganic acids such as hydrochloric acid, hydrobromic acid, hydroiodic acid, sulfuric acid, phosphoric acid, and the like, and organic acids such as *p*-toluenesulfonic, methanesulfonic acid, oxalic acid, *p*-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, hydroiodide, dihydroiodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, $\alpha$-hydroxybutyrate, glycolate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid, mandelic acid, p-toluenesulfonic acid, and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. It should be recognized that the particular counterion forming a part of any salt of this invention is usually not of a critical nature, so long as the salt as a whole is pharmacologically acceptable and as long as the counterion does not contribute undesired qualities to the salt as a whole. It is further understood that such salts may exist as a hydrate.

As used herein, the term "stereoisomer" refers to a compound made up of the same atoms bonded by the same bonds but having different three-dimensional structures which arc not interchangeable. The three-dimensional structures are called configurations. As used herein, the term "enantiomer" refers to two stereoisomers whose molecules are nonsuperimposable mirror images of one another. The term "chiral center" refers to a carbon atom to which four different groups are attached. As used herein, the term "diastereomers" refers to stereoisomers which are not enantiomers. In addition, two diastereomers which have a different configuration at only one chiral center are referred to herein as "epimers". The terms "racemate", "racemic mixture" or "racemic modification" refer to a mixture of equal parts of enantiomers.

The term "enantiomeric enrichment" as used herein refers to the increase in the amount of one enantiomer as compared to the other. A convenient method of expressing the enantiomeric enrichment achieved is the concept of enantiomeric excess, or "ee", which is found using the following equation:

$$ee = \frac{E^1 - E^2}{E^1 + E^2} X 100$$

wherein $E^1$ is the amount of the first enantiomer and $E^2$ is the amount of the second enantiomer. Thus, if the initial ratio of the two enantiomers is 50:50, such as is present in a racemic mixture, and an enantiomeric enrichment sufficient to produce a final ratio of 50:30 is achieved, the ee with respect to the first enantiomer is 25%. However, if the final ratio is 90: 10, the ee with respect to the first enantiomer is 80%. An ee of greater than 90% is preferred, an ee of greater than 95% is most preferred and an ee of greater than 99% is most especially preferred. Enantiomeric enrichment is readily determined by one of ordinary skill in the art using standard techniques and procedures, such as gas or high performance liquid chromatography with a chiral column. Choice of the appropriate chiral column, eluent and conditions necessary to effect separation of the enantiomeric pair is well within the knowledge of one of ordinary skill in the art. In addition, the enantiomers of compounds of Formula I can be resolved by one of ordinary skill in the art using standard techniques well known in the art, such as those described by J. Jacques, et al., "Enantiomers, Racemates, and Resolutions", John Wiley and Sons, Inc., 1981.

The compounds of the present invention have one or more chiral centers and may exist in a variety of stereoisomeric configurations. As a consequence of these chiral centers, the compounds of the present invention occur as racemates, mixtures of enantiomers and as individual enantiomers, as well as diastereomers and mixtures of diastereomers. All such racemates, enantiomers, and diastereomers are within the scope of the present invention.

The terms "R" and "S" are used herein as commonly used in organic chemistry to denote specific configuration of a chiral center. The term "R" (rectus) refers to that configuration of a chiral center with a clockwise relationship of group priorities (highest to second lowest) when viewed along the bond toward the lowest priority group. The term "S" (sinister) refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice", (J.H. Fletcher, et al., eds., 1974) at pages 103-120.

The specific stereoisomers and enantiomers of compounds of Formula I can be prepared by one of ordinary skill in the art utilizing well known techniques and processes, such as those disclosed by Eliel and Wilen, "Stereochemistry of Organic Compounds", John Wiley & Sons, Inc., 1994, Chapter 7. Separation of Stereoisomers. Resolution. Racemization, and by Collet and Wilen, "Enantiomers, Racemates, and Resolutions", John Wiley & Sons, Inc., 1981. For example, the specific stereoisomers and enantiomers can be prepared by stereospecific syntheses using enantiomerically and geometrically pure, or enantiomerically or geometrically enriched starting materials. In addition, the specific stereoisomers and enantiomers can be resolved and recovered by techniques such as chromatography on chiral stationary phases, enzymatic resolution or fractional recrystallization of addition salts formed by reagents used for that purpose.

As used herein the term "Pg" refers to a suitable nitrogen protecting group. Examples of a suitable nitrogen protecting group as used herein refers to those groups intended to protect or block the nitrogen group against undesirable reactions during synthetic procedures. Choice of the suitable nitrogen protecting group used will depend upon the conditions that will be employed in subsequent reaction steps wherein protection is required, and is well within the knowledge of one of ordinary skill in the art. Commonly used nitrogen protecting groups are disclosed in Greene, "Protective Groups In Organic Synthesis," (John Wiley & Sons, New York (1981)). Suitable nitrogen protecting groups comprise acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, alpha.-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, alpha.,.alpha.-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; alkyl groups such as benzyl, triphenylmethyl, benzyloxymethyl and the like; and silyl groups such as trimethylsilyl and the like. Preferred suitable nitrogen protecting groups are formyl, acetyl, methyoxycarbonyl, benzoyl, pivaloyl, t-butylacetyl, phenylsulfonyl, benzyl, t-butyoxycarbonyl (Boc) and benzyloxycarbonyl (Cbz).

As used herein the term "$(C_1-C_4)$alkyl" refers to a straight or branched, monovalent, saturated aliphatic chain of 1 to 4 carbon atoms and includes, but is not limited to methyl, ethyl, n-propyl, isopropyl, n-butyl, isobutyl and the like.

As used herein the term "$(C_1-C_6)$alkyl" refers to a straight or branched, monovalent, saturated aliphatic chain of 1 to 6 carbon atoms and includes, but is not limited to methyl, ethyl, n-propyl, isopropyl, n-butyl, isobutyl, t-butyl, n-pentyl, n-hexyl, and the like.

As used herein, the terms "Me", "Et", "Pr", "iPr", "Bu", "iBu", and "t-Bu" refer to methyl, ethyl, propyl, isopropyl, butyl, isobutyl, and tert-butyl respectively.

As used herein, the terms "Halo", "Halide" or "Hal" refer to a chlorine, bromine, iodine or fluorine atom, unless otherwise specified herein.

The designation " 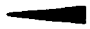 " refers to a bond that protrudes forward out of the plane of the page.

The designation " 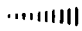 " refers to a bond that protrudes backward out of the plane of the page.

As used herein the term "iGlu$R_5$" refers to the kainate ionotropic glutamate receptor, subtype 5, of the larger class of excitatory amino acid receptors.

As used herein the term "migraine" refers a disorder of the nervous system characterized by recurrent attacks of head pain (which are not caused by a structural brain abnormalitiy such as those resulting from tumor or stroke), gasrointestinal disturbances, and possibly neurological symptoms such as visual distortion. Characteristic headaches of migraine usually last one day and are commonly accompanied by nausea, emesis, and photophobia.

Migraine may represent a "chronic" condition, or an "acute" episode. The term "chronic", as used herein, means a condition of slow progress and long continuance. As such, a chronic condition is treated when it is diagnosed and treatment continued throughout the course of the disease. Conversely, the term "acute"means an exacerbated event or attack, of short course, followed by a period of remission. Thus, the treatment of migraine contemplates both acute events and chronic conditions. In an acute event, compound is administered at the onset of symptoms and discontinued when the symptoms disappear. As described above, a chronic condition is treated throughout the course of the disease.

As used herein the term "patient" refers to a mammal, such a mouse, gerbil, guinea pig, rat, dog or human. It is understood, however, that the preferred patient is a human.

The term "iGlu$R_5$ receptor antagonist" or "iGlu$R_5$ antagonist", as used herein, refers to those excitatory amino acid receptor antagonists which bind to, and antagonize the activity of, the iGlu$R_5$ kainate receptor subtype. As a preferred embodiment, the present invention further provides selective iGlu$R_5$ receptor antagonists. "Selective iGlu$R_5$ receptor antagonist" or "selective iGlu$R_5$ antagonist" as used herein, includes those excitatory amino acid receptor antagonists which selectively bind to, and antagonize, the iGlu$R_5$ kainate receptor subtype, relative to the iGlu$R_2$ AMPA receptor subtype. Preferably, the "selective iGlu$R_5$ antagonists" for use according to the methods of the present invention have a binding affinity at least 10 fold greater for iGlu$R_5$ than for iGlu$R_2$, more preferably at least 100 fold greater. WO 98/45270 provides examples of selective iGlu$R_5$ receptor antagonists and discloses methods for synthesis.

As used herein, the terms "treating", "treatment", or "to treat" each mean to alleviate symptoms, eliminate the causation of resultant symptoms either on a temporary or permanent basis, and to prevent, slow the appearance, or reverse the progression or severity of resultant symptoms of the named disorder. As such, the methods of this invention encompass both therapeutic and prophylactic administration.

As used herein the term "effective amount" refers to the amount or dose of the compound, upon single or multiple dose administration to the patient, which provides the desired effect in the patient under diagnosis or treatment. An effective amount can be readily determined by the attending diagnostician, as one skilled in the art, by the use of known techniques and by observing results obtained under analogous circumstances. In determining the effective amount or dose of compound administered, a number of factors are considered by the attending diagnostician, including, but not limited to: the species of mammal; its size, age, and general health; the degree of involvement or the severity of the disease involved; the response of the individual patient; the particular compound administered; the mode of administration; the bioavailability characteristics of the preparation administered; the dose regimen selected; the use of concomitant medication; and other relevant circumstances.

A typical daily dose will contain from about 0.01 mg/kg to about 100 mg/kg of each compound used in the present method of treatment. Preferably, daily doses will be about 0.05 mg/kg to about 50 mg/kg, more preferably from about 0. 1 mg/kg to about 25 mg/kg.

Oral administration is a preferred route of administering the compounds employed in the present invention whether administered alone, or as a combination of compounds capable of acting as an iGlu$R_5$ receptor antagonist. Oral administration, however, is not the only route, nor even the only preferred route. Other preferred routes of administration include transdermal, percutaneous, pulmonary, intravenous, intramuscular, intranasal, buccal, or intrarectal routes. Where the iGlu$R_5$ receptor antagonist is administered as a combination of compounds, one of the compounds may be administered by one route, such as oral, and the other may be administered by the transdermal, percutaneous, pulmonary, intravenous, intramuscular, intranasal, buccal, or intrarectal route, as particular circumstances require. The route of administration may be varied in any way, limited by the physical properties of the compounds and the convenience of the patient and the caregiver.

The compounds employed in the present invention may be administered as pharmaceutical compositions and, therefore, pharmaceutical compositions incorporating compounds of Formula I are important embodiments of the present invention. Such compositions may take any physical form that is pharmaceutically acceptable, but orally administered pharmaceutical compositions are particularly preferred. Such pharmaceutical compositions contain, as an active ingredient, an effective amount of a compound of Formula I, including the pharmaceutically acceptable salts, prodrugs, and hydrates thereof, which effective amount is related to the daily dose of the compound to be administered. Each dosage unit may contain the daily dose of a given compound, or may contain a fraction of the daily dose, such as one-half or

one-third of the dose. The amount of each compound to be contained in each dosage unit depends on the identity of the particular compound chosen for the therapy, and other factors such as the indication for which it is given. The pharmaceutical compositions of the present invention may be formulated so as to provide quick, sustained, or delayed release of the active ingredient after administration to the patient by employing well known procedures.

Compositions are preferably formulated in a unit dosage form, each dosage containing from about 1 to about 500 mg of each compound individually or in a single unit dosage form, more preferably about 5 to about 300 mg (for example 25 mg). The term "unit dosage form" refers to a physically discrete unit suitable as unitary dosages for a patient, each unit containing a predetermined quantity of active material calculated to produce the desired therapeutic effect, in association with a suitable pharmaceutical carrier, diluent, or excipient.

The inert ingredients and manner of formulation of the pharmaceutical compositions are conventional. The usual methods of formulation used in pharmaceutical science may be used here. All of the usual types of compositions may be used, including tablets, chewable tablets, capsules, solutions, parenteral solutions, intranasal sprays or powders, troches, suppositories, transdermal patches and suspensions. In general, compositions contain from about 0.5% to about 50% of the compounds in total, depending on the desired doses and the type of composition to be used. The amount of the compound, however, is best defined as the "effective amount", that is, the amount of each compound which provides the desired dose to the patient in need of such treatment. The activity of the compounds employed in the present invention do not depend on the nature of the composition, hence, the compositions are chosen and formulated solely for convenience and economy.

Capsules are prepared by mixing the compound with a suitable diluent and filling the proper amount of the mixture in capsules. The usual diluents include inert powdered substances such as starches, powdered cellulose especially crystalline and microcrystalline cellulose, sugars such as fructose, mannitol and sucrose, grain flours, and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

Tablets are often coated with sugar as a flavor and sealant. The compounds may also be formulated as chewable tablets, by using large amounts of pleasant-tasting substances such as mannitol in the formulation, as is now well-established practice. Instantly dissolving tablet-like formulations are also now frequently used to assure that the patient consumes the dosage form, and to avoid the difficulty in swallowing solid objects that bothers some patients.

A lubricant is often necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which swell when wetted to break up the tablet and release the compound. They include starches, clays, celluloses, algins and gums. More particularly, corn and potato starches, methylcellulose, agar, bentonite, wood cellulose, powdered natural sponge, cation-exchange resins, alginic acid, guar gum, citrus pulp and carboxymethylcellulose, for example, may be used, as well as sodium lauryl sulfate.

Enteric formulations are often used to protect an active ingredient from the strongly acid contents of the stomach. Such formulations are created by coating a solid dosage form with a film of a polymer which is insoluble in acid environments, and soluble in basic environments. Exemplary films are cellulose acetate phthalate, polyvinyl acetate phthalate, hydroxypropyl methylcellulose phthalate and hydroxypropyl methylcellulose acetate succinate.

When it is desired to administer the compound as a suppository, the usual bases may be used. Cocoa butter is a traditional suppository base, which may be modified by addition of waxes to raise its melting point slightly. Water-miscible suppository bases comprising, particularly, polyethylene glycols of various molecular weights are in wide use, also.

Transdermal patches have become popular recently. Typically they comprise a resinous composition in which the drugs will dissolve, or partially dissolve, which is held in contact with the skin by a film which protects the composition. Many patents have appeared in the field recently. Other, more complicated patch compositions are also in use, particularly those having a membrane pierced with innumerable pores through which the drugs are pumped by osmotic action.

The following table provides an illustrative list of formulations suitable for use with the compounds employed in the present invention. The following is provided only to illustrate the invention and should not be interpreted as limiting the present invention in any way.

**Formulation 1**

Hard gelatin capsules are prepared using the following ingredients:

| | Quantity (mg/capsule) |
|---|---|
| Active Ingredient | 250 |
| Starch, dried | 200 |
| Magnesium stearate | 10 |
| Total | 460 mg |

The above ingredients are mixed and filled into hard gelatin capsules in 460 mg quantities.

**Formulation 2**

A tablet is prepared using the ingredients below:

| | Quantity (mg/tablet) |
|---|---|
| Active Ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg.

**Formulation 3**

An aerosol solution is prepared containing the following components:

| | Weight % |
|---|---|
| Active Ingredient | 0.25 |
| Ethanol | 29.75 |
| Propellant 22 (Chlorodifluoromethane) | 70.00 |
| Total | 100.00 |

The active compound is mixed with ethanol and the mixture added to a portion of the Propellant 22, cooled to -30°C and transferred to a filling device. The required amount is then fed to a stainless steel container and diluted with the remainder of the propellant. The valve units are then fitted to the container.

**Formulation 4**

Tablets each containing 60 mg of active ingredient are made as follows:

| | |
|---|---|
| Active Ingredient | 60.0 mg |
| Starch | 45.0 mg |
| Microcrystalline cellulose | 35.0 mg |
| Polyvinylpyrrolidone | 4.0 mg |
| Sodium carboxymethyl starch | 4.5 mg |

(continued)

| | |
|---|---|
| Magnesium stearate | 0.5 mg |
| Talc | 1.0 mg |
| Total | 150 mg |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate, and talc, previously passed through a No. 60 mesh U.S. sieve, are then added to the granules which, after mixing, are compressed on a tablet machine to yield tablets each weighing 150 mg.

**Formulation 5**

Capsules each containing 80 mg medicament are made as follows:

| | |
|---|---|
| Active Ingredient | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 sieve, and filled into hard gelatin capsules in 200 mg quantities.

**Formulation 6**

Suppositories each containing 225 mg of active ingredient may be made as follows:

| | |
|---|---|
| Active Ingredient | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

**Formulation 7**

Suspensions each containing 50 mg of medicament per 5 ml dose are made as follows:

| | |
|---|---|
| Active Ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

**Formulation 8**

An intravenous formulation may be prepared as follows:

| | |
|---|---|
| Active Ingredient | 100 mg |
| Mannitol | 100 mg |
| 5 N Sodium hydroxide | 200 ml |
| Purified water to total | 5 ml |

It is understood by one of ordinary skill in the art that the procedures as described above can also be readily applied to a method of treating neurological disorders or neurodegenerative conditions, particularly pain and migraine, comprising administering to a patient an effective amount of a compound of Formula I.

Compounds of Formula I and Formula I(a) can be chemically prepared, for example, by following the synthetic routes set forth in the Schemes below. However, the following discussion is not intended to be limiting to the scope of the present invention in any way. For example, the specific synthetic steps for the routes described herein may be combined in different ways, or with steps from different schemes, to prepare the compounds of Formula I and Formula I(a). All substituents, unless otherwise indicated, are as previously defined. The reagents and starting materials are readily available to one of ordinary skill in the art. For example, certain starting materials can be prepared by one of ordinary skill in the art following procedures disclosed in United States Patents Nos. 5,356,902 (issued October 18, 1994) and 5,446,051 (issued August 29, 1995) and 5,670,516 (issued September 23, 1997) the entire contents, all of which, are herein incorporated by reference. Other necessary reagents and starting materials for the below procedures may be made by procedures which are selected from standard techniques of organic and heterocyclic chemistry, techniques which are analogous to the syntheses of known structurally similar compounds, and the procedures described in the Examples, including novel procedures.

Compounds of Formula I, wherein Z represents an oxygen atom, may be synthesized according to Scheme I.

Scheme I

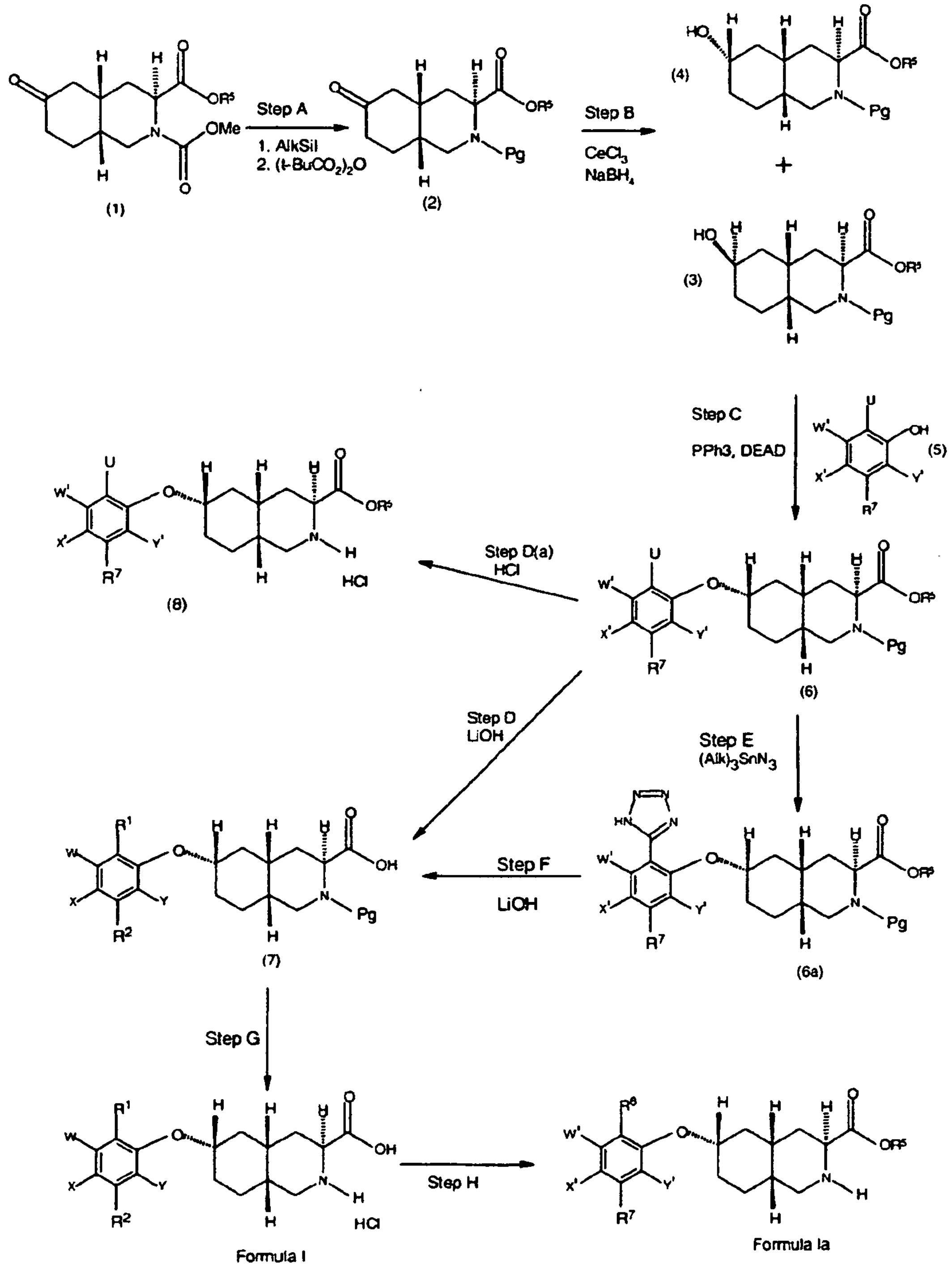


In Scheme I, step A, the compound of structure (1) is treated with a trialkylsilyl iodide ($Alk_3SiI$) and the resulting amine, without isolation, is protected under standard conditions to provide the compound of structure (2). For example, when a protecting group other than methoxycarbonyl is desired, a solution of ethyl-6-oxo-2-methoxycarbonyl-decahydroisoquinolie-3-carboxylate, dissolved in a suitable organic solvent such as dichloromethane at room temperature, is treated with about 4 equivalents of a compound of formula $Alk_3SiI$ such as trimethylsilyl iodide, triethylsilyl iodide, tributylsilyl iodide, and the like, with trimethylsilyl iodide being most preferred. The reaction mixture is stirred for about 10 to 20 hours, quenched with ethanol and concentrated under vacuum. The resulting solid, for example, is then dissolved in a suitable organic solvent such as dichloromethane and treated with an excess of a suitable organic base, such as triethylamine, followed by about 1 equivalent of, for example, di-tert-butyl dicarbonate. The reaction mixture is stirred at

room temperature for 10 to 20 hours. The compound (2) is then isolated using standard procedures. For example, the reaction mixture is concentrated under vacuum, suspended in ethyl acetate and filtered. The filtrate is washed with diluted hydrochloric acid and water, the organic layer separated and dried over anhydrous sodium sulfate, filtered, and concentrated under vacuum to provide concentrated compound (2). Column chromatography may then be performed on silica gel with a suitable eluent such as 25% ethyl acetate/hexane to provide the purified compound (2). Note, where the desired protecting group (Pg) is methoxycarbonyl, the compound of structure (1) may be used directly in Step B below.

In Scheme I, Step B, compound (2) is reduced under standard conditions with a suitable reducing reagent in the presence of a suitable Lewis acid catalyst, to provide the compound of structure (3). For example, ethyl 6-oxo-2-tert-butoxycarbonyl-decahydroisoquinoline-3-carboxylate (compound (2) of Step A above) is mixed with about 1 equivalent of a Lewis acid catalyst, such as cerium trichloride, in a suitable organic solvent such as ethanol. The resulting solution is cooled to -78 °C and about 1 to 2 equivalents of a reducing reagent, such as sodium borohydride, is added and the mixture is warmed slowly to room temperature. After 4 to 8 hours, a suitable acid, such as acetic acid, is added at 0 °C and the resulting mixture is stirred for about 1 to 2 hours at room temperature and concentrated under vacuum. The compound (3) is then isolated using standard procedures such as extraction techniques. For example, the reaction mixture is partitioned between water and an organic solvent such as ethyl acetate, and the aqueous layer is extracted 2-4 times with ethyl acetate. The organic layers are combined, dried over anhydrous sodium sulfate, filtered, and concentrated under vacuum to provide a mixture of compound (3) and compound (4). Both compounds are then purified by chromatography on silica gel with a suitable eluent such as 70% ethyl acetate/hexanes.

In Scheme I, Step C, compound (4) is treated with a compound of structure (5) in the presence of a phosphine and a dialkyl azadicarboxylate to give the compound of structure (6). For example, a solution of ethyl-6-hydroxy-2-tert-butoxycarbonyl-decahydroisoquinoline-3-carboxylate, about 1-1.5 equivalents of compound of structure (5) (wherein U represents hydrogen, CN, $(C_1-C_4)$alkyl-$CO_2R^9$, or $CO_2R^9$) about 0-1.5 equivalents of an organic base such as pyridine, and about 1-1.5 equivalents of a phosphine such as triphenylphosphine in tetrahydrofuran is treated with about 1-1.5 equivalents of a dialkyl azadicarboxylate such as diethyl azodicarboxylate. The reaction is then stirred at 25-70 °C for 15-48 hours. The solvents are removed under vacuum to provide the compound of structure (6). Compound (6) is then purified by chromatography on silica gel with a suitable eluent such as diethyl ether/hexanes or ethyl acetate/ hexanes.

In Scheme I, Step D(a), the compound of structure (6) is deprotected under standard conditions well known in the art to provide the compound of structure (8). For example, compound (6) is treated with an organic solvent such as ethyl acetate saturated with HCl at room temperature for about 3 to 5 hours. The mixture is then concentrated under vacuum to provide the compound of structure (8) wherein U is as defined in Step C. This material may then be purified by techniques well known in the art, such as titration with organic solvents and/or cation exchange chromatography, eluting with MeOH/water, followed by 2N ammonia in MeOH, to provide the purified compound of structure (8) wherein U is as defined in Step C.

In Scheme I, Step D, the compound of structure (6) is hydrolyzed under standard conditions to give the compound of structure (7) wherein $R^1$ is other than tetrazole, but otherwise as defined hereinabove. For example, compound (6) is dissolved in a suitable organic solvent or solvents mixture, such as methanol, ethanol, tetrahydrofuran and/or ethyl acetate, and treated with an excess of a suitable base. Examples of suitable bases include aqueous lithium hydroxide, sodium hydroxide, potassium hydroxide, and the like, with lithium hydroxide being preferred. The reaction is stirred for about 10-36 hours. The reaction mixture is then concentrated under vacuum, diluted with water and washed with ethyl acetate. The aqueous layer is made acidic to pH 3-4 with 10% HCl and extracted with ethyl acetate. These organic phases are combined, dried over sodium sulfate, filtered, and concentrated under vacuum to provide the compound (7) wherein $R^1$ is other than tetrazole, but otherwise as defined hereinabove. The material may then be purified by chromatography on silica gel with a suitable eluent such as ethyl acctate/hexanes/acetic acid, to provide the purified compound.

In Scheme I, where it is desired that the compound of structure (7) contain a tetrazole at $R^1$, compound (6) (wherein U for the purposes of this step is nitrile) is treated with a compound of $Alk_3SnN_3$ in Step E to give the compound of structure (6a). This is followed by hydroysis in Step F, to provide the compound of structure (7) (wherein $R^1$ is tetrazole). For example, compound (6) (wherein U is nitrile) is treated with about 3 to 5 equivalents of azido-tri-n-butyl stannane at about 70 to 100°C for about 12 to 16 hours under an atmosphere of nitrogen to give the compound of structure (6a). Compound (6a) is then hydrolyzed, concentrated, and the resulting compound (7) (wherein $R^1$ is tetrazole) may then be purified, all of which occur under standard conditions well known in the art as described in Step D above.

As an alternative to Steps D and F above, the compounds of structure (6) and (6a) may be selectivley hydrolyzed under standard conditions known in the art to provide a compound of structure (7a):

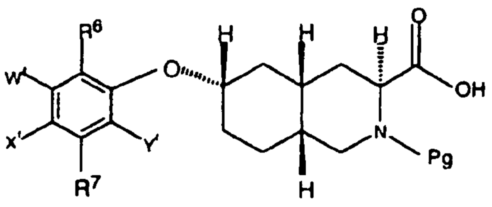

(7a)

For example, compound (6) may be hydrolyzed to provide compounds of (7a) wherein $R^6$ is as defined hereinabove, other than tetrazole, whereas compound (6a) may be hydrolyzed to provide compounds of (7a) wherein $R^6$ is tetrazole. Compound (7a) can then be deprotected under standard conditions to provide the compound of structure (9):

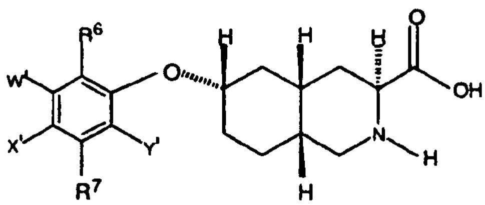

(9)

Methods for the selective hydrolysis of compounds of structure (6) and (6a) are well known in the art.

In Scheme I, Step G, the compound of structure (7) is deprotected under standard conditions well known in the art to provide the compound of Formula I. For example, compound (7) is treated with an organic solvent, such as ethyl acetate, saturated with hydrogen chloride at room temperature for about 3 to 5 hours. The mixture is then concentrated under vacuum to provide the compounds of Formula I. This material may then be purified by techniques well known in the art, such as tritration with organic solvents and/or cation exchange chromatography eluting with methanol/water, followed by 2 N ammonia in methanol, to provide the purified compound of Formula I.

As an alternative to the sequence of Steps D and G, and as an alternative to the sequence of steps F and G, the compounds of structure (6) and (6a), respectively, may be concomitantly hydrolyzed and deprotected as provided below to provide the compounds of Formula I.

Scheme II

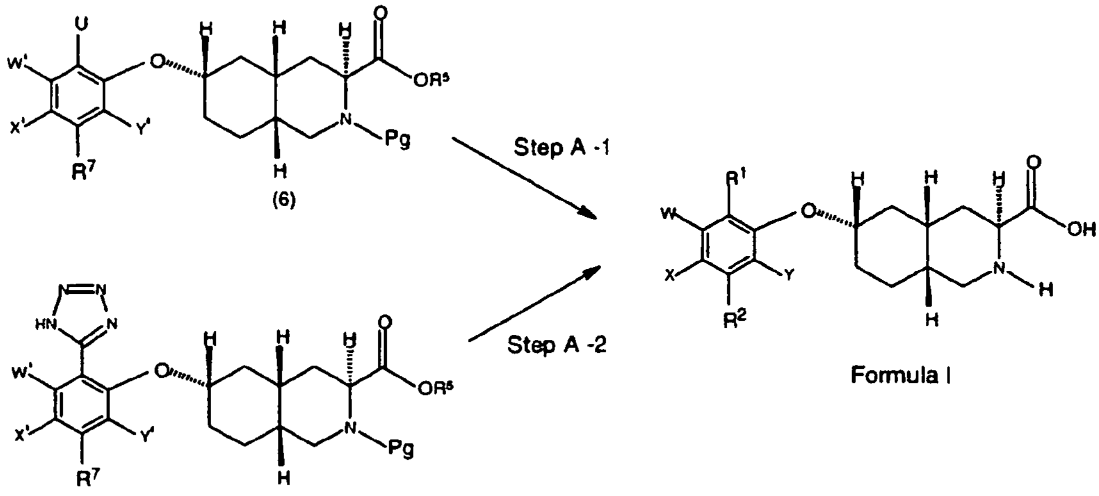


(6)

Formula I

(6a)

In Scheme II, compound (6) or (6a) is deprotected and hydrolyzed concomitantly under standard conditions to provide the compounds of Formula I. For example, in Scheme II, Step A-1, a solution of compound (6), dissolved in 6N HCl, is heated to reflux (90-95 °C) for about 15-20 hours. The reaction mixture is then allowed to cool to room temperature and concentrated in vacuo to provide the compound of Formula I wherein $R^1$ is as defined hereinabove, other than tetrazole. The compound of Formula I can then be purified by techniques well known in the art, such as cation exchange chromatography eluting with methanol/water followed by 2N ammonia in methanol or ethanol to provide the purified compound of Formula I wherein $R^1$ is as defined other than tetrazole. In Scheme II, Step A-2, a solution of compound (6a) is treated as described above in Step A-1 to provide the compound of Formula I wherein $R^1$ is tetrazole. This material may then be purified by techniques well known in the art, such as cation exchange chromatography eluting with methanol/water followed by 2N ammonia in methanol or ethanol to provide the purified compound of Formula I wherein $R^1$ is tetrazole.

In Scheme I. Step H. the compound of Formula I may be nonselectively or selectively esterified to provide the compounds of Formula Ia. For example, the compound of Formula I is dissolved in a suitable organic solvent, such as ethanol, isobutanol, or 2-ethylbutanol, and treated with an excess of a dehydrating agent such as thionyl chloride. The reaction mixture is heated to about 120°C for about 1 to 2 hours. The reaction mixture is then concentrated under vacuum to provide the crude compound of Formula Ia. This material may then be precipitated with diethyl ether and filtered to provide the purified compound. Alternatively in Step H, the compound of Formula I can be esterified by dissolving in a suitable organic solvent such as ethanol, and treating with an excess of a suitable acid. Examples of suitable acids include gaseous hydrochloric acid, aqueous sulfuric acid, p-toluene sulfonic acid, and the like with gaseous hydrochloric acid being preferred. The reaction mixture is heated to reflux (78-85 °C) for about 15-25 hours. The reaction mixture is then concentrated under vacuum to provide the crude compound of Formula Ia. This material can then be purified by techniques well known in the art, such as cation exchange chromatography eluting with methanol/water followed by 2N ammonia in ethanol to provide the purified compound.

Compounds of Formula I, wherein Z represents an oxygen atom and $R^1$ represents tetrazole may alternatively be synthesized according to the procedures set forth in Scheme III.

Scheme III

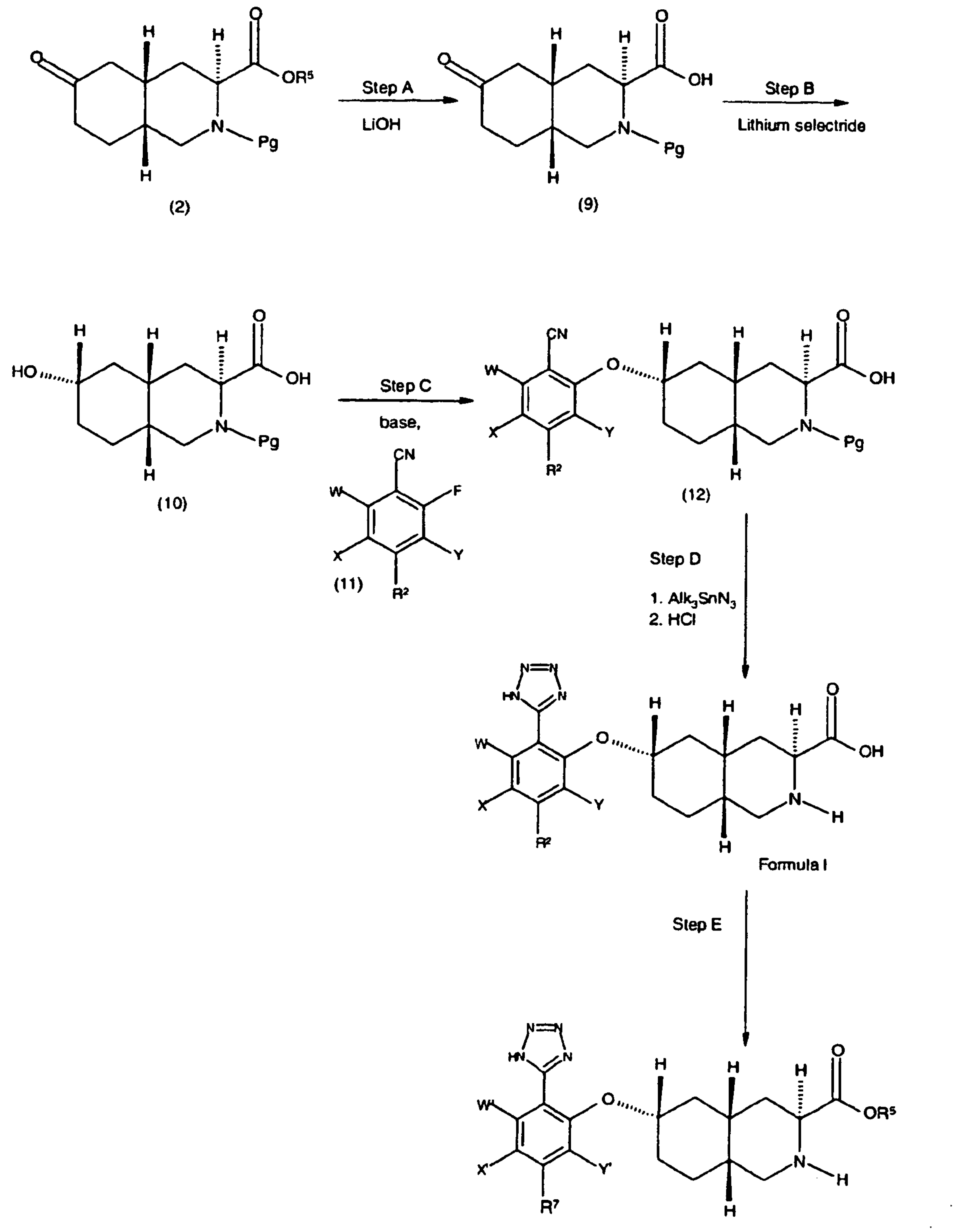


(2) (9) (10) (11) (12) Formula I Formula Ia

In Scheme III, step A, the compound of structure (2) (as previously described in Scheme I above), is hydrolyzed to the compound of structure (9) under standard conditions well known in the art. For example, ethyl 6-oxo-2-(tert-butoxycarbonyl)-decahydroisoquinoline-3-carboxylate is dissolved in a suitable organic solvent or solvents mixture, such

as methanol, ethanol, tetrahydrofuran and/or ethyl acetate, and treated with an excess of a suitable base. Examples of suitable bases include aqueous lithium hydroxide, sodium hydroxide, potassium hydroxide, and the like with lithium hydroxide being preferred. The reaction is stirred for about 20-24 hours at room temperature. The reaction mixture is then concentrated under vacuum, diluted with water and washed with ethyl acetate. The aqueous layer is made acidic to pH 3-4 with 10% HCl and extracted with ethyl acetate. These organic phases are combined, dried over sodium sulfate, filtered, and concentrated under vacuum to provide the compound of structure (9).

In Scheme III, Step B, compound (9) is reduced with a suitable reducing reagent, such as lithium or sodium selectride, to provide the compound of structure (10). For example, 6-oxo-2-tert-butoxycarbonyl-decahydroisoquinoline-3-carboxylic acid is dissolved in a suitable organic solvent such as tetrahydrofuran. The resulting solution is cooled to about 0 °C and about 2 equivalents of lithium selectride, dissolved in tetrahydrofuran, is added. The reaction mixture is warmed slowly to room temperature. After about 2 to 3 hours, a suitable acid, such as I N hydrochloric acid, and sodium chloride are added and the resulting mixture is filtered. The compound (10) is then isolated using standard procedures such as extraction techniques. For example, the aqueous layer is extracted 2-4 times with ethyl acetate. The organic layers are combined, dried over anhydrous sodium sulfate, filtered, and concentrated under vacuum to provide the crude compound (10). Compound (10) may then be purified by chromatography on silica gel with a suitable eluent such as 60% ethyl acetate/hexanes.

In Scheme III, Step C, compound (10) is treated with a compound of structure (11) in the presence of a suitable base to provide the compound of structure (12). For example, a solution of 6-hydroxy-2-tert-butoxycarbonyl-dccahydroisoquinoline-3-carboxylic acid, dissolved in an organic solvent such as tetrahydrofuran, is treated at 0 °C with about 2-2.5 equivalents of a suitable base such as potassium tert-butoxide. The resulting mixture is stirred at room temperature for about 20 to 40 minutes, cooled to 0 °C and treated with about 1-1.5 equivalents of compound (11) (where $R^2$, W, X, and Y are as defined hereinabove). The reaction is then stirred at room temperature for about 15-24 hours. The reaction mixture is then diluted with water and washed with ethyl acetate. The aqueous layer is made acidic to pH 3-4 with 10% HCl and extracted with ethyl acetate. These organic phases are combined, dried over sodium sulfate, filtered, and concentrated under vacuum to provide the compound of structure (12). Compound (12) may then be purified by chromatography on silica gel with a suitable eluent such as ethyl acetate/hexanes

In Scheme III, Step D, compound (12) is treated with a compound of formula $AuC_3SnN_3$, wherein Alk is an alkyl group such as Me, Et, Bu, and the like, and the resulting compound is deprotected without further treatment to the compound of Formula I, wherein $R^1$ is tetrazole, under standard conditions well known in the art. For example, compound (12) is treated with about 3 to 5 equivalents of azido tri-n-butyl stannane at 70 to 100 °C for about 12 to 76 hours under an atmosphere of nitrogen. The mixture is then treated with an organic solvent, such as ethyl acetate, saturated with hydrogen chloride at room temperature for about 3 to 5 hours. The mixture is then concentrated under vacuum to provide the compound of Formula I, wherein $R^1$ is tetrazole. This material may then be purified by techniques well known in the art, such as trituration with organic solvents and/or cation exchange chromatography eluting with methanol/water followed by 2 N ammonia in methanol to provide the purified compound.

In Scheme III, Step E, the compound of Formula I is nonselectively, or selectively, esterified to provide the compound of Formula Ia, wherein $R^6$ is tetrazole. For example, the compound of Formula I is dissolved in a suitable base, such as ethanol, isobutanol, or 2-ethylbutanol, and treated with an excess of a dehydrating agent, such as thionyl chloride. The reaction mixture is heated to 120 °C for about 1-2 hours. The reaction mixture is then concentrated under vacuum to provide the crude compound of Formula Ia, wherein $R^6$ is tetrazole. This material is precipitated with diethyl ether and filtered to provide the purified compound of Formula Ia. Alternatively, in Step E, the compound of Formula I can be esterified by dissolving in a suitable organic solvent such as ethanol, and treating with an excess of a suitable acid. Examples of suitable acids include gaseous hydrochloric acid, aqueous sulfuric acid, p-toluene sulfonic acid, and the like with gaseous hydrochloric acid being preferred. The reaction mixture is heated to reflux (78-85 °C) for about 15-25 hours. The reaction mixture is then concentrated under vacuum to provide the crude compound of Formula Ia. This material can then be purified by techniques well known in the art, such as cation exchange chromatography eluting with methanol/water followed by 2N ammonia in ethanol to provide the purified compound.

One of ordinary skill in the art will understand that the nitrile group of structure (11) (see Scheme III above) can be converted to a protected tetrazole group prior to treatment of compound (10) with compound (11) and, thus, provide yet another route of synthesis for compounds of Formula I wherein $R^1$ is tetrazole. This alternate route is provided in Scheme IV(a) and IV(b) below:

Scheme IV(a)

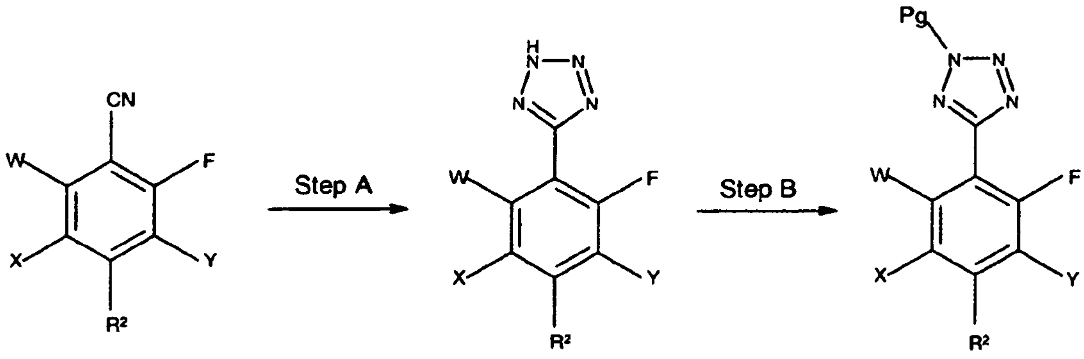


(11) (11a) (11b)

In Scheme IV(a), Step A, the compound of structure (11) is treated under standard conditions to provide the compound of structure (11a). For example, a solution of trimethyl aluminum in toluene is added to a round bottomed flask under nitrogen and the solution cooled to about -7 degrees Celsius. Azidotrimethylsilane (about 3.86 mol) is then added via cannula such that the internal temperature of the reaction is maintained at no greater than about 3 degrees Celsius. To this mixture, the compound of structure (11) is added dropwise in a solution of toluene. The reaction is slowly warmed to RT and then heated to about 90 degrees Celsius. The reaction is heated at 90 degrees for about 13 hours before cooling to RT. The reaction is then cooled to about 0 degrees Celsius in an ice bath, then slowly transferred via cannula to a solution of 6N aqueous HCl and ethyl acetate, pre-cooled to about -5 degrees Celsius. The internal temperature during the quench is maintained at no greater than about 5 degrees. After addition, the flask is allowed to warm to room temperature. The reaction is then diluted with ethyl acetate to dissolve any solids, the layers are separated, and the aqueous layer extracted with ethyl acetate. The organics are combined, washed with brine, dried over anhydrous sodium sulfate, and concentrated using standard techniques well known in the art to provide the concentrated compound of structure (11a)

In Scheme IV(a), Step B, the compound of structure (11a) is protected with a suitable nitrogen protecting group under standard conditions to provide the compound of structure (11b). For example, to a slurry of compound (11a) and 4,4'-dimethoxybezhydrol in glacial acetic acid, is added concentrated sulfuric acid. Upon addition, the reaction becomes red and homogenous and an endotherm of about 3 to 4 degrees Celsius is observed. After about 15 minutes, the product of structure (11b) begins to crystallize, resulting in a slight exotherm of less than about 10 degrees. After about 1 hour, the product is isolated using standard techniques, such as filtration, washed with water, and then with isopropyl alchohol. The product of compound (11b) is dried and concentrated under vaccum to provide the concentrated compound of structure (11b).

Scheme IV(b)

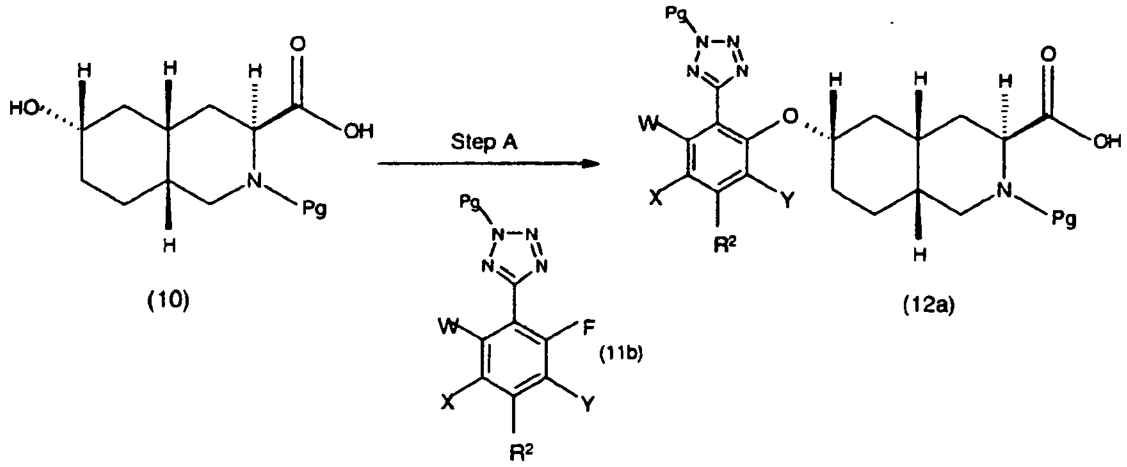


In Scheme IV(b), Step A, the compound of structure (10) (from Scheme III above) is treated with the compound of structure (11b) from Scheme IV(a), Step B above, to provide the compound of structure (12a). For example, to a solution of sodium hydride in dry dimethyl sulfoxide, is added compound (10) dropwise as a solution of dimethyl sulfoxide. During addition, a cooling bath is used to maintain the reaction temperature at or below about 25 degrees Celsius. The reaction is stirred for about 15 minutes at ambient temperature and then compound (11b) is added in one portion as a solid. The reaction slurry is stirred at RT for about 20 minutes before heating to about 40 degrees Celsius for about 2.5 - 3 hours. The reaction is quenched by addition of 1N aqueous HCl solution, water, and ethyl acetate. The layers are separated and the aqueous layer is extracted with ethyl acetate. The combined organics are washed with water and about a 10% solution of aqueous sodium chloride solution. The organic layer is then dried over anhydrous sodium sulfate and concentrated under vaccum to provide the crude compound of structure (12a). This material may then be purified using standard techniques such as chromatography on silica gel, eluting with a suitable eluent such as 1% MeOH in methylene chloride, followed by 5% MeOH in methylene chloride to afford the purified product of compound (12a)

As one of ordinary skill in the art will recognize, the compound of structure (12a) may then be deprotected using, for example TMSI (iodotrimethylsilane) in methylene chloride, to provide the compound of Formula I, wherein $R^1$ is tetrazole. Alternatively, the compound of structure (12a) may be esterified under standard conditions well known in the art, followed by deprotection, to provide the compound of Formula Ia, wherein $R^6$ is tetrazole.

Scheme IV(a) and IV(b), above, provide procedures for the synthesis of Formula I and I(a) compounds where $R^1$ or $R^6$ is tetrazole, wherein the protected hydroxy acid of compound (10) is treated with the protected aryl tetrazole of compound 11(b) in a nucleophilic aromatic substitution reaction to provide the compound of structure 12(a). As discussed, compound 12(a) can then be esterified and/or deprotected, all under standard conditions, to provide the compounds of Formula I(a) or Formula I. Scheme IV(c) provides a general synthetic route for these procedures.

Scheme IV(c)

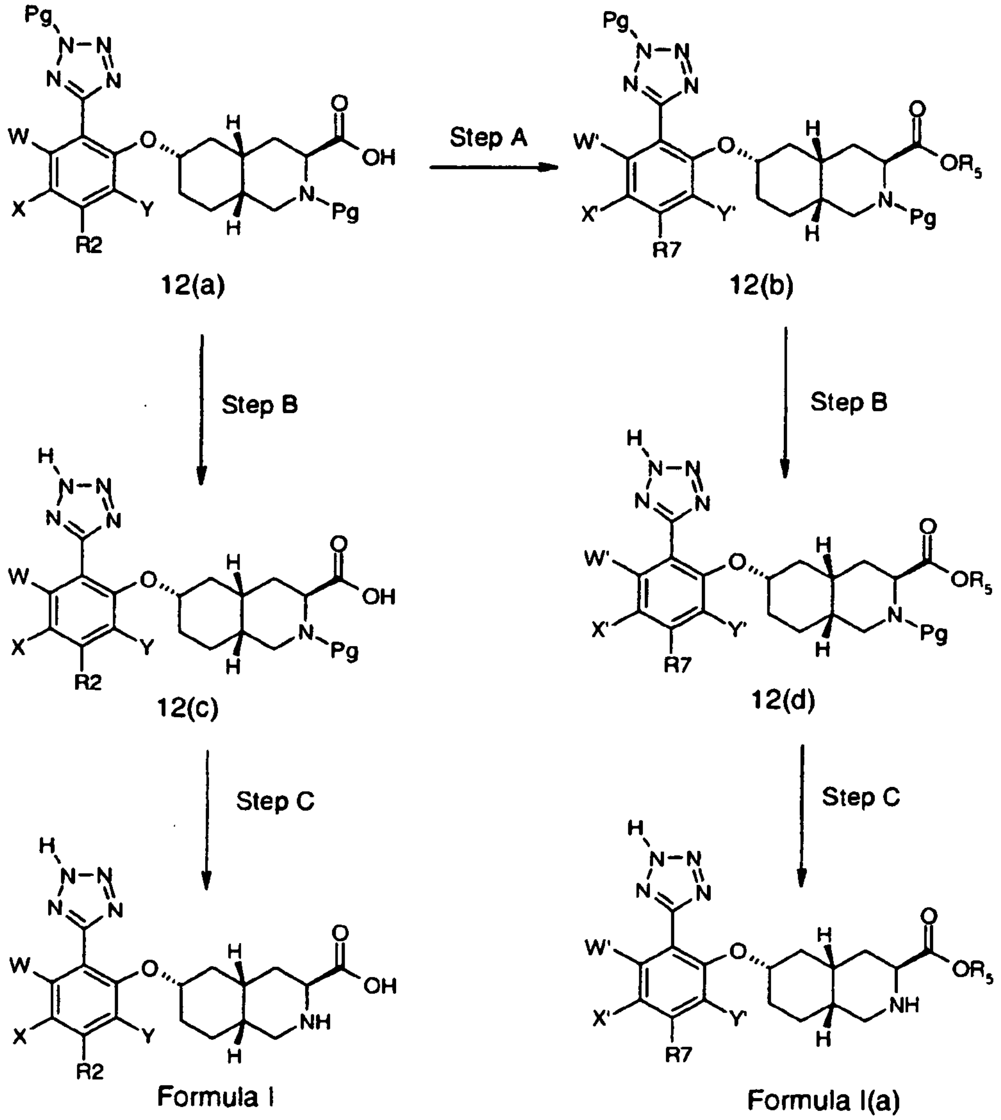


12(a) 12(b) 12(c) 12(d) Formula I Formula I(a)

In Scheme IV(c), Step A, the compound of structure 12(a) is esterified under standard conditions to provide the compound of structure 12(b) wherein $R^6$ is tetrazole. For example, compound 12(a), dissolved in a suitable solvent such as DMF, is treated with a compound of the formula $R^5$-LG where LG represents a suitable leaving group such as a halide. For example $R^5$ is as defined previously and LG represents a chloro or bromo atom. The reaction is heated until complete (confirmed for example by TLC and HPLC). For example, heating at 80°C under nitrogen for about 1hour. The reaction is then cooled to ambient temperature and submitted to standard extractive worhup techniques know to the skilled artisan to provide purified compound 12(b), wherein $R^6$ is tetrazole.

In Scheme IV(c), Steps B and C, the compound of structure 12(a) or 12(b) is deprotected under standard conditions to provide Formula I or Formula I(a) wherein $R^1$ or $R^6$ is tetrazole. For example, to a solution of compound 12(a) or 12(b) is added anisole and trifluoroacetic acid (TFA). The solution is stired until the reaction is complete. For example stirring for 6 hours at room temperature. The product, compound 12(c) or 12(d), can be isolated by standard workup conditions.

Compounds 12(c) and 12(d) are further deprotected in Step C to provide the final products of Formula I or I(a) (wherein $R^1$ or $R^6$ is tetrazole). For example, compound 12(c) or 12(d) is combined with TMSI in $CH_2Cl_2$ and the reaction is stirred until complete. The Final compounds of Formula I or I(a) wherein $R^1$ or $R^6$ is tetrazole may be isolated after standard workup conditions.

Surprisingly, and as a further embodiment of the present invention, Applicants have discovered alternative nucleophilic aromatic substitution procedures for the synthesis of Formula I or Formula I(a) (wherein $R^1$ or $R^6$ is tetrazole) that offer additional advantages over the procedures described in Schemes IV(a) - IV(c). These additional procedures are generally provided in Scheme IV(d), below.

Scheme IV(d)

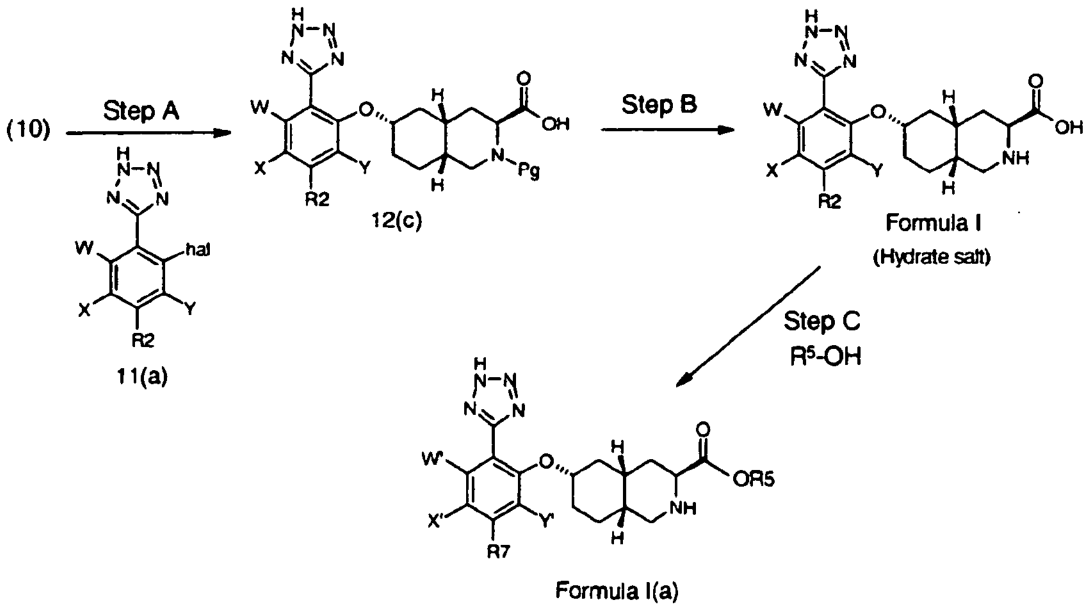


(10)

11(a)

12(c)

Formula I
(Hydrate salt)

Formula I(a)

In Scheme IV(d), Step A, compound (10) is treated with an unprotected aryl tetrazole of structure 11(a) to provide the compound of structure 12(c). For example, to a solution of a suitable base, such as potassium tert-butoxide, in a suitable solvent, such as THF, is added the compound of structure (10) and the unprotected aryl tetrazole of structure 11(a). The reactionis heated until complete. For example, the reaction is heated to about 65°C for about 4 hours. The compound 12(c) is isolated by standard workup techniques.

In Scheme IV(d), Step B, compound 12(c) is deprotected to provide the compound of Formula I wherein $R^1$ is tetrazole. For example, to a solution of a suitable base, such as 85% KOH in water, compound 12(c) is added and the mixture is heated to about 100°C until the reaction is complete. The mixture is cooled then added to a suitable acid, such as HCl, to provide the precipitated acid salt of Formula I, wherein $R^1$ is tetrazole. The acid salt of Formula I ($R^1$ is tetrazole) can purified by standard recrystallization techniques and isolated as the salt or a solvate thereof such as the hydrate.

In Scheme IV(d), Step C, Formula I is esterifed with a compound of the formula $R^5$-OH under standard conditions. For example, a mixture of the hydrate salt of Formula I (Step B above), a suitable acid such as p-toluenesulfonic acid monohydrate, a compound of formula $R^5$-OH, and water is heated to about 140°C until complete. The ester salt of Formula I(a) where R6 is tetrazole is isolated by standard techniques and may be purified by standard recrystallization techniques.

The Formula I compounds of the present invention may be chemically synthesized, for example, from a 6-oxo-2-methoxycarbonyl-decahydroisoquinoline-3-carboxylate intermediate, or a 6-hydroxy-2-tert-butoxycarbonyl-decahydroisoquinoline-3-carboxylate intermediate, or a 6-hydroxy-2-methoxycarbonyl-decahydroisoquinoline-3-carboxylate intermediate. These intermediates, in turn, may be synthesized from a 6-oxo-2-methoxycarbonyl-decahydroisoquinoline-3-carboxylic acid, the synthesis of which is described in United States Patents No. 4,902,695 , No. 5,446,051, and No. 5,356,902 (the contents of which are all herein incorporated by reference). A route for the synthesis of the 6-oxo-2-methoxycarbonyl-dexahydroisoquinoline-3-carboxylate intermediate, useful for the synthesis of the compounds of the present invention, is shown in Scheme VI below. Synthesis of the 6-hydroxy-2-tert-butoxycarbonyl-decahydroisoquinoline-3-carboxylate intermediate is provided in Preparation 1 (infra), while synthesis of the 6-hydroxy-2-methoxycarbonyl-decahydroisoquinoline-3-carboxylate is provided, for example, essentially as described in. Scheme I (Steps A and B) and as provided in United States Patents No. 4,902,695, No. 5,446,051, and No. 5,356,902.

Scheme VI

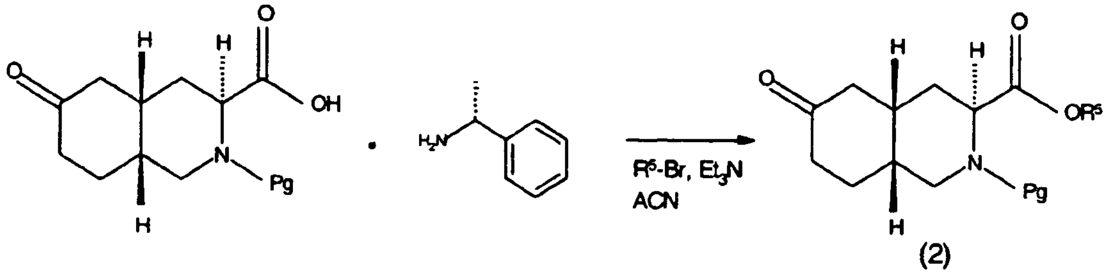

(2)

In Scheme VI, Step A, 6-oxo-2-(Pg)-decahydroisoquinoline-3-carboxylic acid (Pg is as herein defined above) is esterified by reaction with a compound of formula $R^5$-Br (where $R^5$ is as herein defined) to provide the 6-oxo-2-(Pg)-decahydroisoquinoline-3-carboxylate intermediate of compound (2). For example 6-oxo-2-methoxycarbonyl-decahydroisoquinoline-3-carboxylic acid is dissolved in acetonitrile and treated with triethylamine and bromoethane. The reaction is heated at 50°C for about 3 hours, cooled and partitioned between 50:50 ethyl acetate/heptane and 1N HCL. The organic phase is isolated and washed 3 times with water, saturated sodium bicarbonate, brine, dried over anhydrous sodium sulfate, filtered, and concentrated under vacuum to provide ethyl 6-oxo-2-methoxycarbonyl-decahydroisoquinoline-3-carboxylate, a compound of structure (2). This crude material may then be purified under standard conditions well known in the art. For example, the crude material is dissolved in 10% ethyl acetate/heptane and applied to a plug of silica gel (10 g in 10% ethyl acetate/heptane). The plug is eluted with, 10% ethyl acetate/heptane, 15% ethyl acetate/heptane, and 25% ethyl acetate/heptane. The eluents are combined and concentrated under vacuum to provide the purified compound of structure (2).

The following preparations and examples further illustrate the invention and represent typical synthesis of the compounds of Formula I as described generally above. The reagents and starting materials are readily available to one of ordinary skill in the art. As used herein, the following terms have the meanings indicated: "i.v." refers to intravenously; "p.o." refers to orally; "i.p." refers to intraperitoneally; "eq" or "equiv." refers to equivalents; "g" refers to grams; "mg" refers to milligrams; "L" refers to liters; "mL" refers to milliliters; "μL" refers to microliters; "mol" refers to moles; "mmol" refers to millimoles; "psi" refers to pounds per square inch; "mm Hg" refers to millimeters of mercury; "min" refers to minutes; "h" or "hr" refers to hours; "°C" refers to degrees Celsius; "TLC" refers to thin layer chromatography; "HPLC" refers to high performance liquid chromatography; "$R_f$" refers to retention factor; "$R_t$" refers to retention time; "δ" refers to part per million down-field from tetramethylsilane; "THE" refers to tetrahydrofuran; "DMF" refers to N,N-dimethylformamide; "DMSO" refers to dimethyl sulfoxide; "aq" refers to aqueous; "EtOAc" refers to ethyl acetate; "iPrOAc" refers to isopropyl acetate; "MeOH" refers to methanol; "MTBE" refers to tert-butyl methyl ether; "$PPh_3$" refers to triphenylphosphine; "DEAD" refers to diethyl azodicarboxylate; "RT" refers to room temperature; "$K_i$" refers to the dissociation constant of an enzyme-antagonist complex and serves as an index of ligand binding; and "$ID_{50}$" and "$ID_{100}$" refer to doses of an administered therapeutic agent which produce, respectively, a 50 % and 100% reduction in a physiological response.

## Preparation 1

Ethyl (3*S*, *4aS*, 6*R*, 8a*R*) 6-hydroxy-2-*tert*-butoxycarbonyl-1,2,3,4,4a,5,6,7.8,8a-decahydroisoquinoline-3-carboxylate.

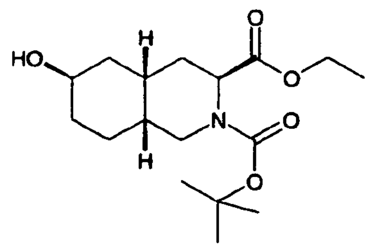

A. Ethyl (3*S*, *4aS*, 6*R*, 8a*R*) 6-hydroxy-2-*tert*-butoxycarbonyl-1,2,3,4,4a,5,6,7,8,8a-decahydroisoquinoline-3-carboxylate.

To a -78 °C solution of (3*S*, 4a*S*, 8a*R*) 6-oxo-2-*tert*-butoxycarbonyl-1,2,3,4,4a,5,6,7,8,8a-decahydroisoquinoline-3-carboxylic acid (6.88 g, 21.14 mmol)(see Preparation 2, Steps A and B, below and Scheme III, Step A generally) and cerium trichloride heptahydrate (7.9 g, 21.2 mmol) in absolute ethyl alcohol (90 mL), under nitrogen sodium borohydride (1.24g, 32.73 mmol) was added in portions. The resulting mixture was slowly warmed up to room temperature over 5 hours. The reaction mixture was cooled down to 0 °C and acetic acid (50 % in water. 25 mL) was carefully added. The resulting mixture was stirred for 1 hour at room temperature and the solvent was removed *in vacuo.* To the resulting material water and ethyl acetate were added and the phases separated. Aqueous layer was extracted with ethyl acetate (3X). The combined organic phases were dried, filtered and concentrated *in vacuo*. Flash chromatography (silicagel, 70% ethyl acetate/hexane) gave ethyl (3*S*, 4a*S*, 6*S*, 8a*R*) 6-hydroxy-2-*tert*-butoxycarbonyl-1,2,3,4,4a,5,6,7,8,8a-decahydroisoquinoline-3-carboxylate (2.4 g, 35%) and the title compound (4.15 g, 60%)
Ion Electrospray Mass Spectrum M+Na: 350.2

**<u>Preparation 2</u>**

(3*S*, 4*aS*, 6*S*, 8a*R*) 6-hydroxy-2-*tert*-butoxycarbonyl-1,2,3,4,4a,5,6,7,8,8a-decahydroisoquinoline-3-carboxylic acid.

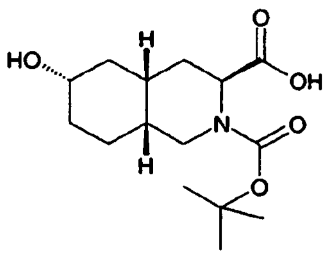

A. Ethyl (3*S*, 4*aS*, 8a*R*) 6-oxo-2-*tert*-butoxycarbonyl-1,2,3,4,4a,5,6,7,8,8a-decahydroisoquinoline-3-carboxylate.

To a solution of ethyl (3*S*, 4*aS*, 8a*R*) 6-oxo-2-methoxycarbonyl-1,2,3,4,4a,5,6,7,8,8a-decahydroisoquinoline-3-carboxylate (3.54 g, 12.5 mmol) in methylene chloride (100mL) under nitrogen, iodotrimethylsilane (10.0g, 50 mmol) was added in one portion at room temperature. The reaction mixture was stirred overnight and quenched with ethanol (20-30 mL). The solution was concentrated *in vacuo* and dried for 3 hours under reduced pressure. The resulting solid was dissolved in methylene chloride (100 mL) and triethylamine (7mL, 50 mmol) was added. After stirring for 15 minutes a solution of di-tert-butyl-dicarbonate (2.73g, 12.5 mmol) in methylene chloride(10 mL) was added. The resulting mixture was stirred overnight at room temperature and concentrated *in vacuo.* The resulting solid was suspended in ethyl acetate and filtered. The filtrate was washed with 1N hydrochloric acid and brine. The organic phase was dried, filtered and concentrated *in vacuo.* Flash chromatography (silicagel, 25% ethyl acetate/hexane) gave pure product as an colorless oil (3.69 g, 92%).

B. (3*S*, 4*aS*, 8aR) 6-oxo-2-*tert*-butoxycarbonyl-1,2,3,4,4a,5,6,7,8,8a-decahydroisoquinoline-3-carboxylic acid.

To a solution of ester from preparation 2A (9.7 g, 29.81 mmol) in absolute ethanol (130 mL) a solution of lithium hydroxide (2.5 N, 132 mL) was added. The resulting mixture was stirred at room temperature for 22 h. The ethanol was removed *in vacuo* and the resulting mixture was washed with ethyl acetate (x2). The aqueous phase was made acidic by addition of 10 % hydrochloric acid (pH= 3-4) and extracted with ethyl acetate (x3). The resulting organic phases were combined, dried and concentrated *in vacuo* to afford the title compound (8.85 g, 100%)

C. (3*S*, 4a*S*, 6*S*, 8a*R*) 6-hydroxy-2-*tert*-butoxycarbonyl-1,2,3,4,4a,5,6,7,8,8a-decahydroisoquinoline-3-carboxylic acid.

To an ice-cooled solution of ketone from preparation 2B (11.34g, 38.14 mmol) in dry tetrahydrofuran (50 mL) under nitrogen a solution of L-Selectride (1 M in tetrahydrofuran, 76 mL) was added dropwise. The resulting mixture was allowed to reach room temperature for 2 h and quenched with 1N hydrochloric acid (78 mL). To the resulting mixture sodium chloride was added to saturate the aqueous phase. After filtration the aqueous phase was extracted with ethyl

acetate. The combined organic phases were dried and concentrated *in vacuo.* Flash chromatography (silicagel, ethyl acetate-hexane 4:3) afforded the desired alcohol (8.5 g, 74%)

**Example 9**

Preparation of (3*S*, 4a*S*, 6*S*, 8a*R*) 6-[3-Chloro-2-(1(2)*H*-tetrazol-5-yl)-phenoxyl-1,2,3,4,4a,5,6,7,8,8a-decahydroisoquinoline-3-carboxylic acid hydrochloride

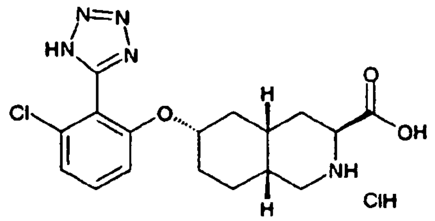

A. Preparation of (3*S*, *4aS*, 6*S*, 8a*R*) 6-(3-chloro-2-cyano-phenoxy)-2-*tert*-butoxycarbonyl-1,2,3,4,4a,5,6,7,8,8a-decahydroisoquinoline-3-carboxylic acid.

To an ice-cooled solution of the material from preparation 2 (400 mg, 1.34 mmol) in dry tetrahydrofuran (5.6 mL) under nitrogen a solution of potassium *tert*-butoxide (1M in tetrahydrofuran, 3.0 mL) was slowly added. The resulting suspension was stirred at room temperature for 25 min and again cooled to 0-5 °C before the addition of 2-chloro-6-fluorobenzonitrile (249 mg, 1.60 mmol). The reaction mixture was stirred a room temperature overnight, diluted with water and washed with ethyl acetate (x2). The aqueous phase was made acidic (pH= 3-4) with 10 % hydrochloric acid and extracted with ethyl acetate (x2). The resulting organic phases were combined, dried and concentrated *in vacuo*. Flash chromatography (silicagel, 75% ethyl acetate/hexane/2.5 % acetic acid) gave the desired compound as a white solid (460 mg, 79%).
Ion Electrospray Mass Spectrum M+1-*t*-butylOCO: 335.2

B. Preparation of (3*S*, 4a*S*, 6*S*, 8a*R*) 6-(3-chloro-2-(1(2)*H*-tetrazol-5-yl)-phenoxy]-2-*tert*-butoxycarbonyl-1,2,3,4,4a, 5,6,7,8,8a-decahydroisoquinoline-3-carboxylic acid.

A mixture of the nitrile from Example 9, step A (3.03 g, 6.9 mmol) and azidotri-n-butylstannane (7.6 mL, 28 mmol) was stirred under nitrogen at 85 °C for 60 h. The mixture was diluted with ethyl acetate (20 mL) and sodium hydroxide (2.5N, 25 mL) was added. The resulting mixture was stirred for 1 h. The aqueous layer was washed with ethyl acetate (2X) and concentrated *in vacuo*. Flash chromatography (silicagel, 55% ethyl acetate/hexane/1 % acetic acid) gave the desired tetrazol as an oil (1.18 g, 35%).
Ion Electrospray Mass Spectrum M+1: 478.1

C. Preparation of (3*S*, 4a*S*, 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylic acid hydrochloride

A suspension of the protected decahydroisoquinoline from step B (400 mg, 0.84 mmol) in ethyl acetate saturated with hydrogen chloride (5 mL) was stirred for 4 h at room temperature. The mixture was extracted with water (1X). The aqueous layer was washed with ethyl acetate (2X) and concentrated *in vacuo* to afford a white solid (320 mg, 92%).
Ion Electrospray Mass Spectrum M-HCl+1: 378.1
$^1$H NMR ($CD_3OD$, 200.13 MHz): 7.54 (t, J = 8.3 Hz, 1 H); 7.22 (t, J = 8.1 Hz, 2H); 4.47 (m, 1 H); 4.00 (dd, J = 12.6, 3.5 Hz, 1 H); 3.21 (t, J = 12.6 Hz, 1 H); 3.06 (dd, J = 12.7, 4.7 Hz, 1 H); 2.21-1.59 (m, 10 H).

**Example 10**

Preparation of 2-Ethyl-butyl (3*S*, *4aS*, 6*S*, 8a*R*) 6-[3-chloro-2-(1(2)*H*-tetrazol-5-yl)-phcnoxy]-1,2,3,4,4a, 5,6,7,8,8a-decahydroisoquinoline-3-carboxylate hydrochloride

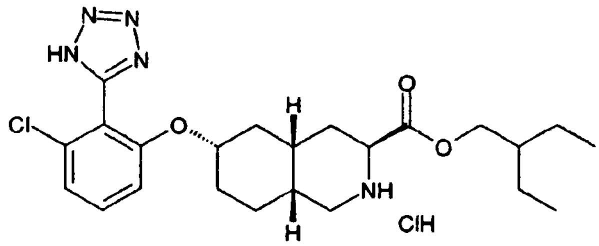

To a suspension of the compound from Example 9, step C (800 mg, 2.18 mmol) in 2-ethylbutanol (50 mL), thionyl chloride (1.75 mL, 24 mmol) was added dropwise. The resulting solution was stirred at 120 °C for 2 h. The solvent was removed *in vacuo* and diethyl ether was added. The resulting solid was filtered and washed with diethyl ether) to give the desired material (711 mg, 70%).
Ion Electrospray Mass Spectrum M-HCl+1: 462.3
$^1$H NMR ($CD_3OD$, 200.13 MHz): 7.53 (t, J = 8.0 Hz, 1 H); 7.24 (d, J = 8.2 Hz, 1 H); 7.19 (d, J = 8.0 Hz, 1 H); 4.45 (br s, 1 H); 4.22 (dd, J = 10.8, 5.7 Hz, 1 H); 4.14 (m, 2 H); 3.23 (t, J = 12.6 Hz, 1 H); 3.11 (d, J = 9.6 Hz, 1 H); 2.19-1.36 (m, 15 H); 0.91 (t, J = 7.5 Hz, 6 H).

## Example 12

Preparation of (3*S*, *4aS*, *6S*, 8a*R*) 6-[3-fluoro-2-(1(2)*H*-tetrazol-5-yl)-phenoxy]-1,2,3,4,4a,5,6,7,8,8a-decahydroisoquinoline-3-carboxylic acid hydrochloride

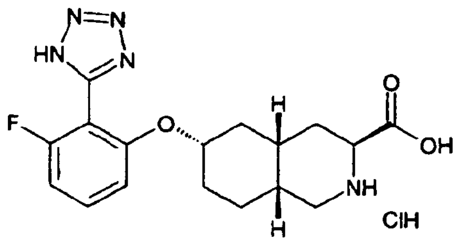

A. Preparation of (3*S*, *4aS*, 6*S*, 8a*R*) 6-(3-fluoro-2-cyano-phenoxy)-2-*tert*-butoxycarbonyl-1,2,3,4,4a,5,6,7,8,8a-decahydroisoquinoline-3-carboxylic acid.

Following the procedures as described in Example 9,step A, material from preparation 2 (300 mg, 1 mmol) in tetrahydrofuran (4.2 mL) was treated with a solution of potassium *tert*-butoxide (1 M in tetrahydrofuran, 2.2 mL) and 2,6-difluorobenzonitrile (209 mg, 1.5 mmol) to give 377 mg of the title compound (90%).
Ion Electrospray Mass Spectrum M+Na: 441.2

B. Preparation of (3*S*, 4a*S*, 6*S*, 8a*R*) 6-[3-fluoro-2-(1(2)*H*-tetrazol-5-yl)-phenoxyl-2-*tert*-butoxycarbonyl-1,2,3,4,4a, 5,6,7,8.8a-decahydroisoquinoline-3-carboxylic acid.

Following the procedures as described in Example 9, step B, compound from step A (370 mg, 0.88 mmol) was treated with azidotri-n-butylstannane (1.0 mL, 3.6 mmol) at 90 °C for 31 h to give the desired compound (120 mg, 29%).
Ion Electrospray Mass Spectrum M+1: 462.3

C. Preparation of (3*S*, *4aS*, 6*S*, 8a*R*) 6-[3-fluoro-2-(1(2)*H*-tetrazol-5-yl)-phenoxy]-1,2,3,4,4a,5,6,7,8,8a-decahydroisoquinoline-3-carboxylic acid hydrochloride.

Following the procedures as described in Example 9, step C, material from step B (120 mg, 0.26 mmol) treated with ethyl acetate saturated with hydrogen chloride (3 mL) gave the desired aminoacid (60 mg, 64%).
Ion Electrospray Mass Spectrum M-HCl+1: 362.2
$^1$H NMR ($CD_3OD$, 200.13 MHz): 7.57 (dt, J = 8.3, 6.7 Hz, 1 H); 7.13 (d, J = 8.9 Hz, I H); 6.97-6.88 (m, 1 H); 4.59-4.49

(m, 1 H); 4.05 (dd, J = 12.1, 4.3 Hz, 1 H); 3.38-3.25 (m, 1 H); 3.11 (dd, J = 12.9, 4.6 Hz, 1 H); 2.27-1.39 (m, 10 H).

**Preparation 10**

(3*S*, *4aS*, 6*S*, 8a*R*) 6-hydroxy-2-methoxycarbonyl-1,2,3,4,4a,5,6,7,8,8a-decahydroisoquinoline-3-carboxylic acid.

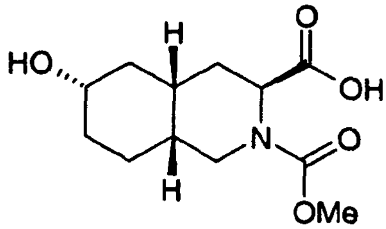

To a slurry of 17.3 g of (3*S*, 4a*S*, 8a*R*) 6-oxo-2-methoxycarbonyl-1,2,3,4,4a,5,6,7,8,8a-decahydroisoquinoline-3-carboxylic acid (46 mmol) in 240 mL of tetrahydrofuran is added 106 mL if 1.0 M L-Selectride in tetrahydrofuran. The mixture is stirred for 1 hour at room temperature, then quenched with 130 mL of 1.4 M hydrochloric acid. The layers are separated and the aqueous phase extracted with 50 mL of tert-butyl methyl ether. The organic phases are combined and extracted with 75 mL of saturated aqueous sodium carbonate. The aqueous phase is washed twice with 30 mL portions of tert-butyl methyl ether then acidified to pH 1 with 6 M hydrochloric acid. The product is extracted into 75 mL of tert-butyl methyl ether. The organic mixture is dried with sodium sulfate, and concentrated to 8.17 g of yellow solid. The product is crystallized from 20 mL of tert-butyl methyl ether to provide 6.92 g of the title compound (59% yield).

**Preparation 11**

6-Chloro-2-fluoro-benzotetrazole

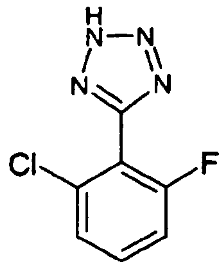

To a 12L round bottomed flask under nitrogen was added trimethyl aluminum (1930 mL of a 2M solution in toluene, 3.86 mol). The flask is cooled to -7 °C before adding azidotrimethylsilane (512.5 mL, 3.86 mol) via canula such that the internal temperature is maintained at less than 3 °C. To this flask is added 6-chloro-2-fluorobenzonitrile (500 g, 3.21 mol) dropwise as a solution in toluene (1L). The reaction is slowly warmed to room temperature then heated to 90 °C. A cold finger is used to condense tetramethylsilane as it boiled from the reaction mixture. The reaction is heated at 90 °C for 13 hours before cooling to room temperature. The reaction is cooed to 0 °C with an ice bath then transferred slowly via cannula to a pre-cooled (-5 °C) solution of 6N aqueous HCl (3L) and ethyl acetate (3L). The internal temperature during the quench is kept at less than 5 °C. After addition, the flask is allowed to warm to room temperature. The reaction is diluted with ethyl acetate (2 L) to disolve solids before tranferring to a 22L flask. The layers are separated and the aqueous layer was extracted with ethyl acetate (1L). The combined organic layers are washed with brine (2L) dried over anhydrous sodium sulfate, and concentrated. 636.4 g. of the title compound is obtained (93% yield). Analysis by HPLC and $^1$H NMR analysis shows the purity as greater than 98%.

**Preparation 12**

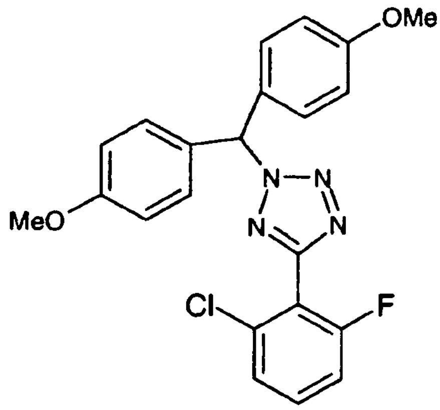

To a thick slurry of the material from Preparation 11, above, (101.8 g, .513 mol) and 4,4'-dimethoxybezhydrol (125 g, .513 mol) in 510 mL glacial acetic acid is added concentrated sulfuric acid (5.5 mL). Upon addition, the reaction immediately becomes red and homogeneous. The red color rapidly lightens to orange over several minutes. An endotherm of 3-4 °C is also observed as the reaction becomes homogeneous. After approximately 15 minutes, the product begins to crystallize from the reaction mixture resulting in a mild exotherm (<10 °C). After 1 hour, the solid is isolated by filtration and washed with water (1L) then isopropyl alcohol (.5 L). The resulting white solid is dried in *vacuo* at 50 °C to afford 199.8 g of the title compound (91 % yield).

**Example 37**

2-Ethyl-butyl (3*S*, 4a*S*, 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate

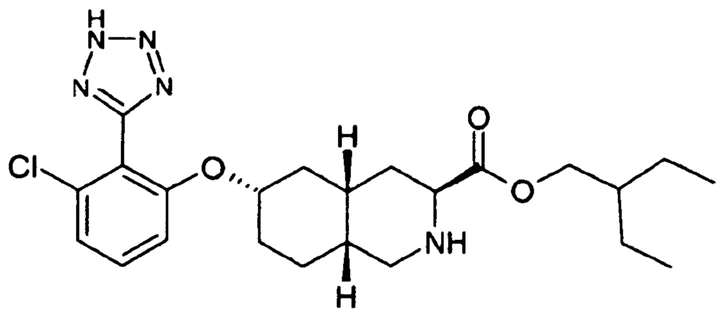

p-toluenesulfonic acid

A. Preparation of

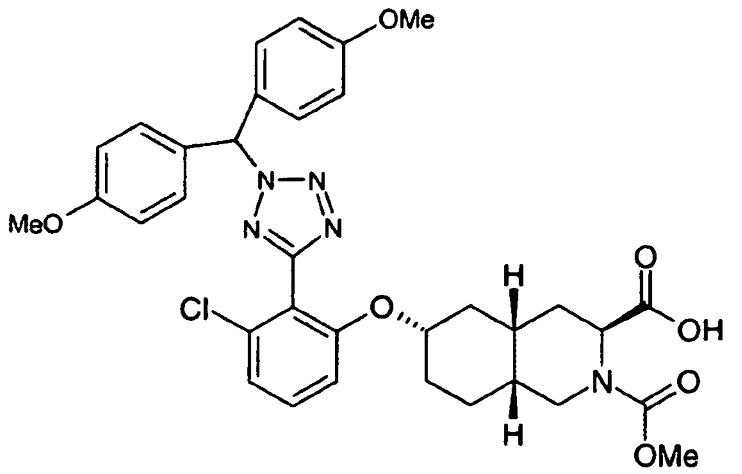

To a slurry of sodium hydride (60% dispersion in mineral oil, 2.94 g, 73.5 mmol) in dry dimethyl sulfoxide (25 mL) is added the material from Preparation 10 (9.44 g, 36.7 mmol) dropwise as a solution in dimethyl sulfoxide (21 mL). During the course of the addition (40 min.), a cooling bath is used to maintain the temperature at or below 25 °C. After stirring 15 minutes at ambient temperature, the material from Preparation 12 (10.4 g, 24.5 mmol) is added in one portion as a solid. The slurry is stirred at room temperature for 20 minutes before heating to 40 °C for 2.75 hours. Analysis of the reaction mixture by HPLC shows no additional progress after this point and the reaction is cooled to room temperature. The reaction is quenched by addition of IN aqueous hydrogen chloride solution (50 mL), water (200 mL) and ethyl actetate (200 mL). The layers are separated and the aqueous layer is extracted with ethyl acetate (1x50 mL). The combined organic layers are washed with water (2x 100 mL) and 10% aqueous sodium chloride solution (1x100 mL). The organic layer is then dried over anhydrous sodium sulfate and concentrated *in vacuo* to afford a crude oil. The crude product is purified on silica gel eluting I % methanol in methylene chloride followed by 5% methanol in methylene chloride to afford 11.84 g. of the title compound (73% yield) as a white foam.

B. Preparation of

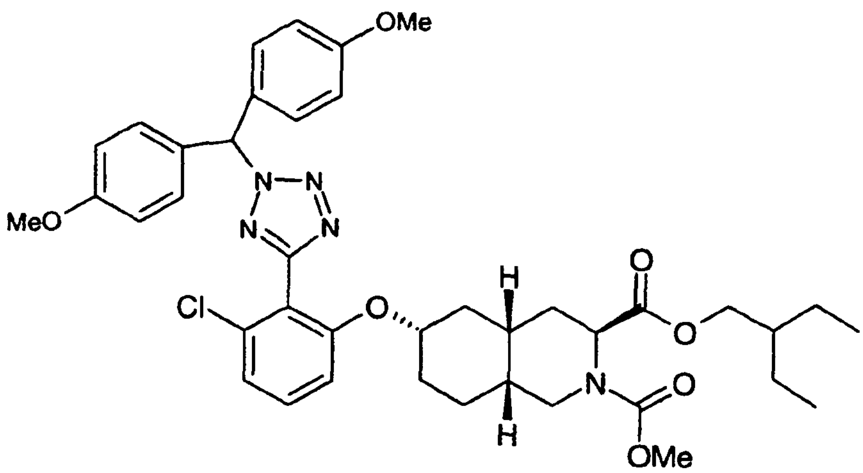

To a solution material from Step A, of the above (16.87 g, 25.4 mmol), in N,N-dimethylformamide (170 mL) is added powder potassium carbonate (4.55 g, 33 mmol) and 3-(bromomethyl)pentane (4.62 mL, 33 mmol). The reaction mixture is heated to 80 °C under nitrogen. After 1 h, analysis by thin layer chromatography and HPLC indicates that the reaction was complete. The reaction is cooled and diluted with water (500 mL) and methylene chloride (170 mL). The organic layer is washed with brine, dried over anhydrous magnesium sulfate, and concentrated in vacuo to afford 17.8

g. of the title compound (94% yield) as a foam.

C. 2-Ethyl-butyl (3*S*, *4aS,* 6*S*, 8a*R*) 6-[3-chloro-2-(1(2)*H*-tetrazol-5-yl)-phenoxy]-2-methoxycarbonyl-1,2,3,4,4a,5,6,7,8,8a-decahydroisoquinoline-3-carboxylate

To a solution of the material from Step B, above (216 g, 289 mmol), is added anisole (94.4 mL, 868.3 mmol) and trifluoroacetic acid (564 mL). The dark red solution is stirred at RT until complete conversion is observed by HPLC (6.5 h). The reaction is concentrated to a dark red oil. The residual trifluoroacetic acid is removed by azeotropic distillation with chloroform. The resulting oil is dissolved in methylene chloride (800 mL) and washed with pH 4 buffer (4x 1 L). The organic layer is dried over anhydrous magnesium sulfate, filtered, and concentrated to a crude oil. The product is purified on silica gel (2 Kg) eluting methylene chloride, 10% ethyl acetate in methylene chloride, then ethyl acetate. The appropriate fractions are concentrated in vacuo to provide 132 g. of the title compound as a white foam (88% yield).

D. 2-Ethyl-butyl (3*S*, 4a*S,* 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate

The material from Step C, above (390 g, 750 mmol) and 187 mL of trimethylsilyliodide are combined in 1.65 L of dichloromethane and the mixture stirred at ambient temperature for 24 hours. At this time the mixture is quenched with 10 mL of saturated aqueous sodium hydrogencarbonate and combined with 325 mL of tetrahydrofuran. The reaction is further quenched with a total of 2.0 L of saturated aqueous sodium hydrogencarbonate. The mixture is stirred for 1 hour and the product is isolated by filtration. The solids are washed with water and dichloromethane then dried in *vacuo* to provide 230 g of the title compound.

The isolated solids are further purified by recrystallization. A mixture of 245 g of the title compound is combined with 2.0 L of water and 200 mL of acetonitrile. The mixture is adjusted to pH 6.8 by the addition of 1.0 M hydrochloric acid. An additional 1.0 L of acetonitrile is added and the mixture is heated to effect a solution. The solution is allowed to cool to ambient temperature producing a precipitate. After the mixture cools to approximately 25 oC for 20 minutes the solids are collected by filtration. The solids are washed with water and dried *in vacuo* to provide 211 g of the title compound.

E. 2-Ethyl-butyl (3*S*, 4a*S,* 6*S,* 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate

To a suspension of the material from Step D, above (211 g, 457 mmol) in 1100 mL of 2-propanol, is added p-toluenesulfonic acid monohydrate (91.7 g, 480 mmol). The mixture is heated to 55 °C to effect a solution then allowed to cool to ambient temperature for 2 hours and then further cooled to approximately 3 °C for 90 minutes. The solids are collected by filtration and washed with 500 mL of cold 2-propanol. The solids are dried *in vacuo* to provide 270 g (93% yield) of the final title compound as a white powder.
$^1$H NMR, 500 MHz dmso-d6, 9.23 (bs, 1H), 8.94 (bs, 1H), 7.56 (t, 1H), 7.45 (d, 2H), 7.30 (d, 1H), 7.24 (d. 1H), 7.09 (d, 2H), 4.37 (m, 1H), 4.17 (m, 1H), 4.13 (dd, 1H), 4.03 (dd, 1H), 3.04 (m, 1H), 2.94 (m, 1H), 2.26 (s, 3H), 2.07 (m, 1H), 1.96 (m, 1H), 1.84 (m, 2H), 1.75 (m, 2H), 1.60 (m, 3H), 1.48 (m, 1H), 1.29 (m, 4H), 1.09 (m, I H), 0.83 (t, 6H)
Mp = 204 °C

**<u>Example 38</u>**

2-Ethyl-butyl (3*S*, 4a*S*, 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate

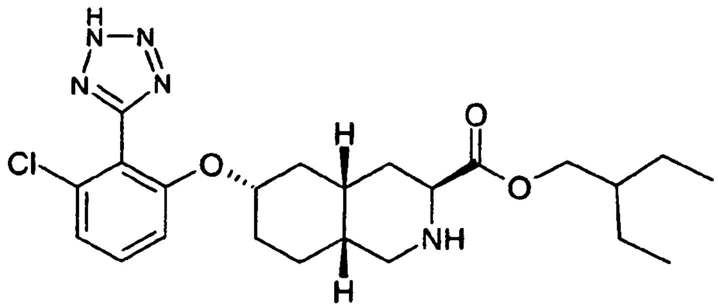

p-toluenesulfonic acid

A. (3*S*, 4a*S*, 6*S*, 8a*R*) 6-[3-chloro-2-(1(2)*H*-tetrazol-5-yl)-phenoxy]-2-methoxycarbonyl-1,2,3,4,4a,5,6,7,8,8a-decahydroisoquinoline-3-carboxylic acid

The material from Preparation 10 (20.0 g, 77.7 mmol) is added to 311 mL of 1 M potassium *tert*-butoxide in tetrahydrofuran followed by the addition of 6-Chloro-2-fluorobenzotetrazole (Preparation 11) (17.0 g, 85.7 mmol). The mixture is heated to 63 °C and monitored for consumption of the aryl tetrazole by HPLC. After 4 hours the reaction is cooled to 20 °C and quenched by the addition of trimethylsilyl chloride (16.9 g, 155.5 mmol), followed by heating to reflux for 30 minutes. The reaction mixture is again cooled to ambient temperature and further quenched by the addition of 224 mL of 1.5 M hydrochloric acid. The organic phase is washed with two 50 mL portions of saturated aqueous sodium chloride. The organic phase is then exchanged for ethyl acetate by atmospheric distillation of the tetrahydrofuran with concurrent addition of ethyl acetate until the distillation temperature reaches 75 °C. The exchange of solvents effects the precipitation of the desired product. The mixture is cooled to 10 °C and the solids are collected by filtration, washed with ethyl acetate, and dried to provide 43.4 g of product as a white solid.

B. (3*S*, 4a*S,* 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylic acid • hydrochloride

To a solution prepared from 85% potassium hydroxide (21.2 g 321 mmol) and 100 mL of water is added the material from Step A, above (20.0 g, 45.9 mmol). The solution is heated to 103 °C for 26 hours at which time the HPLC analysis shows less than 4% starting material remains. The reaction is cooled to 30 °C and is added over 20 minutes to 62 mL of 6 M hydrochloric acid effecting the precipitation of the desired product. The mixture is cooled to 10 °C and the product is collected by filtration, washed with 1 M hydrochloric acid, followed by a wash with acetonitrile. The product is dried in *vacuo* to provide 17 g of the title compound (89% yield) as a white solid.

The title compound (20.0 g) is then combined with 200 mL of water and heated to 90 °C to effect a solution. The solution is cooled to 60 °C. and 40 mL of 6 M hydrochloric acid is added effecting the precipitation of the title compound. The mixture is stirred at 60 °C for 45 minutes then cooled to ambient temperature. The recrystallized hydrate product is collected by filtration and washed with 100 mL of acetonitrile. The product was dried *in vacuo* to 17 g of 2049266 as a white solid and used in the next step

C. 2-Ethyl-butyl (3*S*, 4a*S,* 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate

A mixture of the hydrate from Step B, above(30.0 g, 69.4 mmol), paratoluenesulfonic acid monohydrate (15.84 g, 83.3 mmol), 75.0 mL of 2-ethyl-1-butanol, and 6.0 mL of water is heated to 140 °C over a two hour period. During the heating process, a distillate is collected which contains 11 mL of aqueous and 14 mL of organic phases. The mixture is cooled 70 °C and 75 mL of 2-propanol is added. The mixture is further cooled to 50 °C and 150 mL of *tert*-butyl methyl ether is added over 20 minutes effecting the precipitation of the product. The mixture is then cooled to ambient temperature. The solids are collected by filtration, washed with 50 mL of *tert*-butyl methyl ether, and dried *in-vacuo* to provide 39.0 g (88.6% yield) of the final title compound as a white solid.

A suspension of 10.0 g of the product, in a mixture of 49.0 mL of 2-propanol and 1.0 mL of water, was heated to solution. The solution is allowed to cool to ambient temperature effecting crystallization. The mixture is stirred for 1 hour at less than 29 °C and then the solids are collected by filtration. The solids are washed with 7 mL of 2-propanol and dried *in-vacuo* to provide 8.73 g of the recrystallized title compound.

$^1$H NMR, 500 MHz dmso-d6, 9.23 (bs, 1H), 8.94 (bs, 1H), 7.56 (t, 1H), 7.45 (d, 2H), 7.30 (d, 1H), 7.24 (d, 1H), 7.09 (d,

2H). 4.37 (m, 1H), 4.17 (m, 1H), 4.13 (dd, 1H), 4.03 (dd, 1H), 3.04 (m, 1H), 2.94 (m, 1H), 2.26 (s, 3H), 2.07 (m, 1H), 1.96 (m, 1H), 1.84 (m, 2H), 1.75 (m, 2H), 1.60 (m, 3H), 1.48 (m, 1H), 1.29 (m, 4H), 1.09 (m, 1H), 0.83 (t, 6H).
Mp = 204 °C

## Example 39

To establish that the $iGluR_5$ receptor subtype is mediating a pharmacological response in a neurological disease or disorder, the binding affinity of the panel compounds to the $iGluR_5$ receptor is first measured using standard methods. For example, the activity of compounds acting at the $iGluR_5$ receptor can be determined by radiolabelled ligand binding studies at the cloned and expressed human iGluR5 receptor (Korczak et al., 1994, Recept. Channels 3; 41-49), and by whole cell voltage clamp electrophysiological recordings of currents in acutely isolated rat dorsal root ganglion neurons (Bleakman et al., 1996, Mol. Pharmacol. 49; 581-585). The selectivity of compounds acting at the $iGluR_5$ receptor subtype can then be determined by comparing antagonist activity at the $iGluR_5$ receptor with antagonist activity at other AMPA and kainate receptors. Methods useful for such comparison studies include: receptor-ligand binding studies and whole-cell voltage clamp electrophysiological recordings of functional activity at human $GluR_1$, $GluR_2$,$GluR_3$ and $GluR_4$ receptors (Fletcher et al., 1995, Recept. Channels 3; 21-31); receptor-ligand binding studies and whole-cell voltage clamp electrophysiological recordings of functional activity at human $GluR_6$ receptors (Hoo et al., Recept. Channels 2;327-338); and whole-cell voltage clamp electrophysiological recordings of functional activity at AMPA receptors in acutely isolated cerebellar Purkinje neurons (Bleakman et al., 1996, Mol. Pharmacol. 49; 581-585) and other tissues expressing AMPA receptors (Fletcher and Lodge, 1996, Pharmacol. Ther. 70; 65-89).

iGluR5 antagonist binding affinity profiles

Cell lines (HEK293 cells) stably transfected with human iGluR receptors are employed. Displacement of $^3$[H] AMPA by increasing concentrations of antagonist is measured on $iGluR_1$ , $iGluR_2$, $iGluR_3$, and $iGluR_4$ expressing cells, while displacement of $^3$[H] kainate (KA) is measured on $iGluR_5$, $iGluR_6$, $iGluR_7$, and KA2-expressing cells. Estimated antagonist binding activity ($K_i$) in $\mu$M, for example, is determined for Compounds of Formula I. As an indicia of selectivity, the ratio of binding affinity to the $iGluR_2$ AMPA receptor subtype, versus the binding affinity to $iGluR_5$ kainate receptor subtype ($K_i$ at $iGluR_2$ / $K_i$ at iGluR5) is also determined. The iGluR5 receptor antagonist compounds, as provided by the present invention, provide a $K_i$ at the iGluR5 receptor subtype of less than 5000$\mu$M , preferably less than 500$\mu$M , even more preferably less than 50$\mu$M, and most preferably less than 5$\mu$M. The preferred selective iGluR5 receptor antagonists compounds, as provided by the present invention, display a greater binding affinity (lower $K_i$ ) for $iGluR_5$ than that for $iGluR_2$ , preferably at least 10 fold greater for $iGluR_5$ than that for $iGluR_2$, and even more preferably at least 100 fold, and most preferably at least 1000 fold. than that for $iGluR_2$.

## Example 40

The following animal model may be employed to determine the ability of each of the compounds of Formula I to inhibit protein extravasation, an exemplary functional assay of the neuronal mechanism of migraine.

Animal Model of Dural Protein Extravasation

Harlan Sprague-Dawley rats (225-325 g) or guinea pigs from Charles River Laboratories (225-325 g) are anesthetized with sodium pentobarbital intraperitoneally (65 mg/kg or 45 mg/kg respectively) and placed in a stereotaxic frame (David Kopf Instruments) with the incisor bar set at -3.5 mm for rats or -4.0 mm for guinea pigs. Following a midline sagital scalp incision, two pairs of bilateral holes are drilled through the skull (6 mm posterially, 2.0 and 4.0 mm laterally in rats; 4 mm posteriorly and 3.2 and 5.2 mm laterally in guinea pigs, all coordinates referenced to bregma). Pairs of stainless steel stimulating electrodes, insulated except at the tips (Rhodes Medical Systems, Inc.), are lowered through the holes in both hemispheres to a depth of 9 mm (rats) or 10.5 mm (guinea pigs) from dura.

The femoral vein is exposed and a dose of the test compound is injected intravenously (i.v.) at a dosing volume of 1ml/Kg or, in the alternative, test compound is administered orally (p.o) via gavage at a volume of 2.0ml/Kg. Approximately 7 minutes post i.v. injection, a 50 mg/Kg dose of Evans Blue, a fluorescent dye, is also injected intravenously. The Evans Blue complexes with proteins in the blood and functions as a marker for protein extravasation. Exactly 10 minutes post-injection of the test compound, the left trigeminal ganglion is stimulated for 3 minutes at a current intensity of 1.0 mA (5 Hz, 4 msec duration) with a Model 273 potentiostat/ galvanostat (EG&G Princeton Applied Research).

Fifteen minutes following stimulation, the animals are euthanized by exsanguination with 20 mL of saline. The top of the skull is removed to facilitate the collection of the dural membranes. The membrane samples are removed from both hemispheres, rinsed with water, and spread flat on microscopic slides. Once dried, the tissues are coverslipped

with a 70% glycerol/water solution.

A fluorescence microscope (Zeiss) equipped with a grating monchromator and a spectrophotometer is used to quantify the amount of Evans Blue dye in each sample. An excitation wavelength of approximately 535 nm is utilized and the emission intensity at 600 nm is determined. The microscope is equipped with a motorized stage and also interfaced with a personal computer. This facilitates the computer-controlled movement of the stage with fluorescence measurements at 25 points (500 mm steps) on each dural sample. The mean and standard deviation of the measurements are determined by the computer.

The extravasation induced by the electrical stimulation of the trigeminal ganglion has an ipsilateral effect (i.e. occurs only on the side of the dura in which the trigeminal ganglion was stimulated). This allows the other (unstimulated) half of the dura to be used as a control. The ratio ("extravasation ratio") of the amount of extravasation in the dura from the stimulated side, over the amount of extravasation in the unstimulated side, is calculated. Control animals dosed with only with saline, yield an extravasation ratio of approximately 2.0 in rats and apprximately 1.8 in guinea pigs. In contrast, a compound which completely prevents the extravasation in the dura from the stimulated side would yield a ratio of approximately 1.0.

Dose-response curves are generated for each of the compounds of Formula I and the dose that inhibits the extravasation by 50% ($ID_{50}$) or 100% ($ID_{100}$) is approximated.

## Example 41

To demonstrate the utility of compounds of the present invention to treat pain or provide analgesic effects, several well known animal models may be employed. For example, international application WO 98/45270 describes the well known Formalin Test, which is described below:

### Formalin Test

For example, male Sprague-Dawley rats (200-250g; Charles River,Portage, MI) are housed in group cages and maintained in a constant temperature and a 12 hour light/12 hour dark cycle 4-7 days before studies are performed. Animals have free access to food and water at all times prior to the day of the experiment.

Drugs or vehicles are administered intraperitoneally (i.p.) or orally (p.o.) by gavage in a volume of about 1 ml/kg. The test is performed in custom made Plexiglas® boxes about 25 x 25 x 20 cm in size (according to Shibata et al., Pain 38;347-352, 1989, Wheeler-Aceto et al., Pain, 40; 229-238,1990). A mirror placed at the back of the cage allows the unhindered observation of the formalin injected paw. Rats are acclimated individually in the cubicles at least 1 hour prior to the experiment. All testing is conducted between, for example, 08:00 and 14:00 h and the testing room temperature is maintained at about 21-23°C.

Test compounds are administered about 30 minutes prior to the formalin injection. Formalin (50 micoliters of a 5% solution in saline) is injected subcutaneously into the dorsal lateral surface of the right hind paw with a 27 gauge needle. Observation is started immediately after the formalin injection. Formalin-induced pain is quantified by recording, for example, in 5 minute intervals, the number of formalin injected pawlicking events and the number of seconds each licking event lasts. These recordings are made for about 50 minutes after the formalin injection.

Several different scoring parameters have been reported for the formalin test. The total time spent licking and biting the injected paw is demonstrated to be most relevant (Coderre et al., Eur. J. Neurosci. 6; 1328-1334, 1993; Abbott et al., Pain, 60; 91-102, 1995) and may be chosen for the testing score. The early phase score is the sum of time spent licking, in seconds, from time 0 to 5 minutes. The late phase is scored in 5 minute blocks from 15 minutes to 40 minutes and is expressed accordingly or also by adding the total number of seconds spent licking from minute 15 to minute 40 of the observation period.

Data may be presented as means with standard errors of means (± SEM). Data may also be evaluated by one-way analysis of variance (ANOVA) and the appropriate contrasts analyzed by Dunnett "t" test for two sided comparisons. Differences are considered to be significant if, for example, the P-value is less than 0.05. Statistics may be determined at the 5 minute time point and at 5 minute intervals between 15 and 40 minutes. Where data are expressed as total amount of time spent licking in the late phase, statistics may be performed on the total time spent licking as well and may be indicated accordingly.

In addition to the Formalin Test, the well known Mouse Writhing Test, essentially as described in published International Application WO 00/028980, may also be employed to demonstrate the analgesic properties of compounds of the present invention.

### Mouse Writhing Test

An accepted procedure for detecting and comparing the analgesic activity of different classes of analgesic

drugs, for which there is a good correlation with human analgesic activity, is the prevention of acetic acid-induced writhing in mice. Mice are orally administered various doses of a test compound or placebo prior to testing. The mice are then injected intraperitoneally with acetic acid (0.55% solution, 10 mL/kg) five minutes prior to a designated observation period. Inhibition of writhing behavior is demonstrative of analgesic activity. Haubrich et al., "Pharmacology of pravadoline: a new analgesic agent", The Journal of Pharmacology and Experimental Therapeutics, 255 (1990) 511-522. For scoring purposes "writhe" is indicated by whole body stretching or contracting of the abdomen during an observation period beginning about five minutes after receiving the acetic acid.

$ED_{50}$ values, and their standard error of means (SEM), are determined using accepted numerical methods for all test compounds administered. For example, see R.E. Kirk (1982) "Experimental Design: Procedures for the behavioral sciences," 2nd ed. One method to establish the significance of the analgesic activity of a given test compound compared to that of another is to calculate the SEM values for each $ED_{50}$ value. If the SEM values do not overlap the line of addition, then the ED50 values are significantly different from the line of addition.

Yet another accepted animal model to demonstrate the ability of a particular compound to treat pain, or provide analgesic effects, is the well known Rat Model of Carrageenan-induced Thermal Hyperalgesia, also described in published International Application WO 00/028980.

<u>Carrageenan-induced Thermal Hyperalgesia in Rats</u>

Another accepted method for detecting and comparing the analgesic activity of different classes of analgesic compounds for which there is good correlation with human analgesic activity is the reversal of carrageenan-induced thermal hyperalgesia in rats (Hargreaves et al. Pain 32:77-88, 1988).

Rats are administered a dose test compound or vehicle and then injected subcutaneously into one hindpaw, with carrageenan (1.5% w/v, 100 μl). The response to noxious thermal stimulus is determined two hours later using a commercially available thermal plantar device (Ugo Basil, Italy) according to established methods (Hargreaves et al. Plain 32:77-88, 1988). Briefly, animals are habituated to a plastic behavioral enclosure for 5 min. A heat source is positioned directly beneath a hindpaw and the time taken for hindpaw withdrawal monitored automatically. If the animal does not respond within 20 sec, the stimulus is automatically terminated to prevent tissue damage. Measurements for both the injured and contralateral (control) hindpaw are recorded. Thermal hyperalgesia is evidenced by a shorter response latency by the injured as compared to the control paw.

$ED_{50}$ values and their standard error of means (SEM) are determined using accepted numerical methods. For example, see R.E. Kirk (1982) "Experimental Design: Procedures for the behavioral sciences," 2nd ed.

**Claims**

1. A compound of the formula

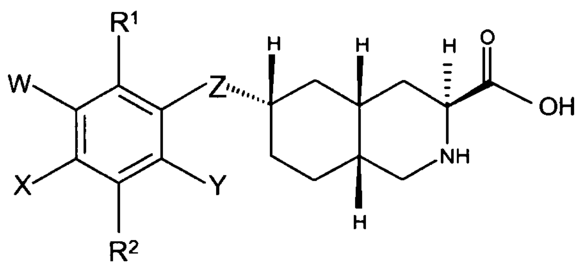

wherein

Z represents an oxygen atom;
$R^1$ represents tetrazole;
$R^2$ represents hydrogen;
W represents halo; and
X, and Y each independently represent hydrogen;
or a pharmaceutically acceptable salt thereof.

2. The compound according to Claim 1 wherein W is Cl.

**3.** A compound according to Claim 1 or 2 which is (3*S*, 4a*S*, 6*S*, 8a*R*) 6-[3-Chloro-2-(1(2)*H*-tetrazol-5-yl)-phenoxy]-1,2,3,4,4a,5,6,7,8,8a-decahydroisoquinoline-3-carboxylic acid or a pharmaceutically acceptable salt thereof.

**4.** The compound according to Claim 3 wherein the pharmaceutically acceptable salt is the hydrochloride salt.

**5.** A compound of the Formula:

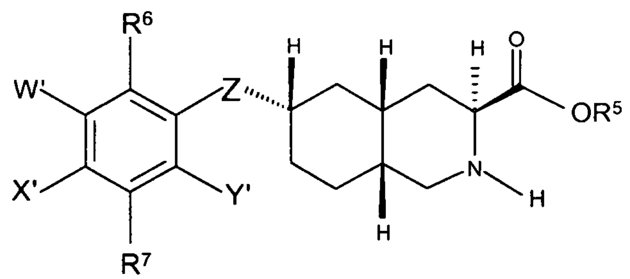

wherein,

Z represents an oxygen atom;
$R^5$ represents $(C_1-C_6)$alkyl;
$R^6$ represents tetrazole;
$R^7$ represents hydrogen;
W' represents halo; and
X' and Y' each independently represent hydrogen;

or a pharmaceutically acceptable salt thereof.

**6.** The compound according to Claim 5 wherein W' represents Cl.

**7.** A compound according to Claim 5 or 6 which is 2-Ethyl-butyl (3*S*, 4a*S*, 6*S*, 8a*R*) 6-[3-chloro-2-(1(2)*H*- tetrazol-5-yl)-phenoxy]-1,2,3,4,4a,5,6,7,8,8a-decahydroisoquinoline-3-carboxylate or a pharmaceutically acceptable salt thereof.

**8.** The compound of Claim 7 wherein the pharmaceutically acceptable salt is the hydrochloride salt.

**9.** A compound according to any one of Claims 1 to 4 for use in therapy.

**10.** A compound according to any one of Claims 5 to 8 for use in therapy.

**11.** A compound which is 2-Ethyl-butyl (3*S*, 4a*S*, 6*S*, 8a*R*) 6-[3-chloro-2-(1(2)*H*-tetrazol-5-yl)-phenoxy]-1,2,3,4,4a, 5,6,7,8,8a-decahydroisoquinoline-3-carboxylate para-toluenesulfonate for use in therapy.

**12.** A compound according to any one of Claims 1 to 4 for use in the treatment of a neurological disorder or neurodegenerative disease.

**13.** A compound according to any one of Claims 5 to 8 for use in the treatment of a neurological disorder or neurodegenerative disease.

**14.** The compound according to Claim 12 or Claim 13 wherein the neurological disorder is pain or migraine.

**15.** A compound according to Claim 1 or Claim 5 for use in the treatment of pain.

**16.** A compound according to Claim 3 for use in the treatment of pain.

**17.** A compound according to Claim 7 for use in the treatment of pain.

**18.** A compound which is 2-Ethyl-butyl (3*S*, 4a*S*, 6*S*, 8a*R*) 6-[3-chloro-2-(1(2)*H*-tetrazol-5-yl)-phenoxy]-1,2,3,4,4a, 5,6,7,8,8a-decahydroisoquinoline-3-carboxylate para-toluenesulfonate for use in the treatment of pain.

**19.** The use of a compound according to any one of Claims 1 to 4 for the manufacture of a medicament for treating a neurological disorder or neurodegenerative disease.

**20.** The use of a compound according to any one of Claims 5 to 8 for the manufacture of a medicament for treating a neurological disorder or neurodegenerative disease.

**21.** The use according to Claim 19 or Claim 20 wherein the neurological disorder is pain or migraine.

**22.** The use of a compound according to Claim 1 or Claim 5 for the manufacture of a medicament for treating pain.

**23.** The use of a compound according to Claim 3 for the manufacture of a medicament for treating pain.

**24.** The use of a compound according to Claim 7 for the manufacture of a medicament for treating pain.

**25.** The use of of a compound which is 2-Ethyl-butyl (3*S*, 4a*S*, 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate for the manufacture of a medicament for treating pain.

**26.** A pharmaceutical composition comprising an effective amount of the compound according to any one of Claims 1 to 4, in combination with a pharmaceutically acceptable carrier, diluent, or excipient.

**27.** A pharmaceutical composition comprising an effective amount of the compound according to any one of Claims 5 to 8, in combination with a pharmaceutically acceptable carrier, diluent, or excipient.

**28.** A pharmaceutical composition comprising an effective amount of the compound which is 2-Ethyl-butyl (3*S*, 4a*S*, 6*S*, 8a*R*) 6-[3-chloro-2-(1(2)*H*-tetrazol-5-yl)-phenoxy]-1,2,3,4,4a,5,6,7,8,8a-decahydroisoquinoline-3-carboxylate para-toluenesulfonate, in combination with a pharmaceutically acceptable carrier, diluent, or excipient.

**29.** A process for preparing a compound of the formula:

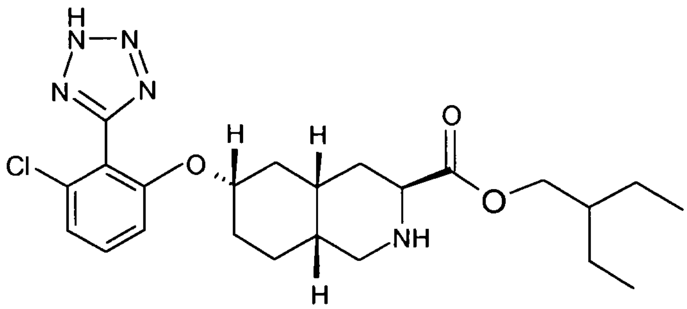

comprising combining a compound of the structure:

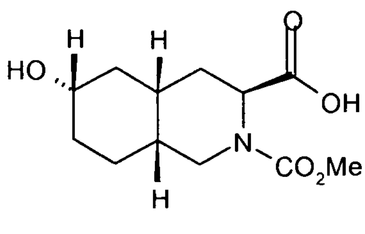

with a suitable base in a suitable solvent, followed by addition of a compound of the structure:

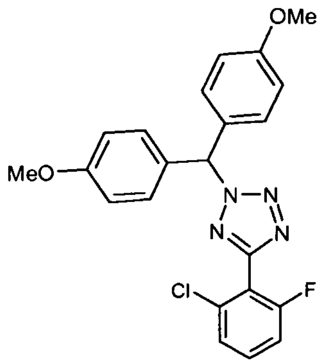

followed by esterification to a compound of the structure:

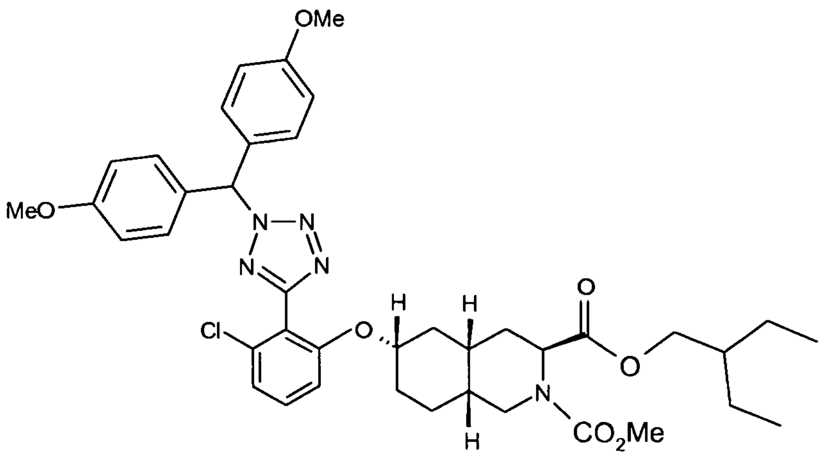

followed by removal of the nitrogen protecting groups, and precipitation with a suitable acid.

**30.** A process for synthesizing a compound of the Formula:

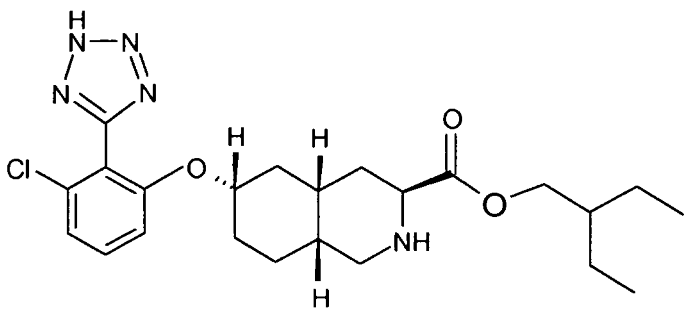

comprising combining a compound of the structure:

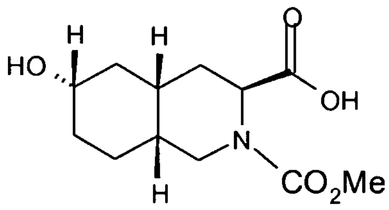

with a suitable base in a suitable solvent, followed by addition of a compound of the structure:

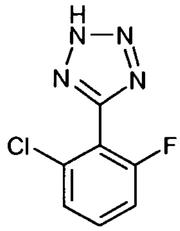

followed by deprotection of the nitrogen group, precipitation with a suitable acid, and crystallization of the hydrate salt of the structure:

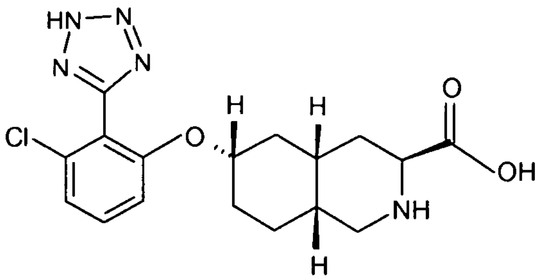

followed by treatment with a suitable alcohol in the presence of a suitable acid to effect the one step esterification and crystallization of the compound of the formula:

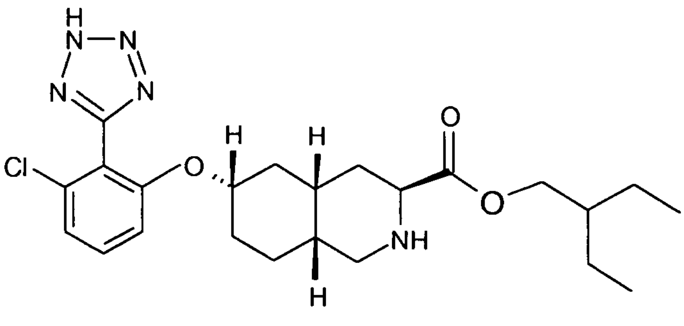

**31.** A process of preparing 2-Ethyl-butyl (3*S*, 4a*S*, 6*S,* 8a*R*) 6-[3-Chloro-2-(1(2)*H*-tetrazol-5-yl)-phenoxy]-1,2,3,4,4a, 5,6,7,8,8a-decahydroisoquinoline-3-carboxylate para-toluenesulfonate, comprising combining a compound of structure:

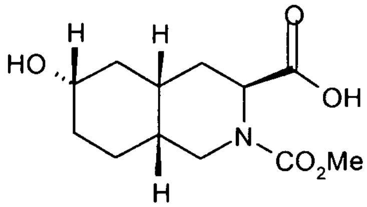

with a suitable base in a suitable solvent, followed by the addition of a compound of structure:

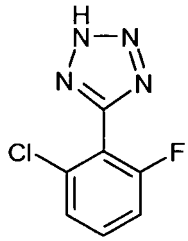

followed by deprotection of the nitrogen protecting group, precipitation with HCl, and crystallization of the monohydrate hydrochloride salt of the compound of the structure:

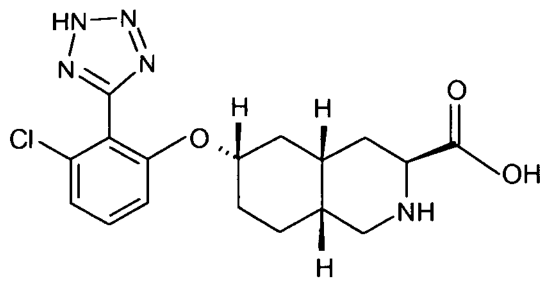

.

followed by treatment with a 2-ethyl-1-butanol in the presence of paratoluenesulfonic acid to effect the one step esterification and crystallization of 2-Ethyl-butyl (3*S*, 4a*S*, 6*S*, 8a*R*) 6-[3-Chloro-2-(1(2)*H*-tetrazol-5-yl)-phenoxy]-1,2,3,4,4a,5,6,7,8,8a-decahydroisoquinoline-3-carboxylate para-toluenesulfonate .

**Patentansprüche**

**1.** Verbindung der Formel

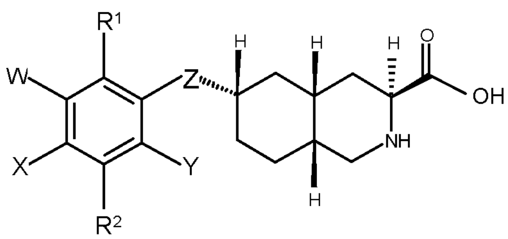

worin

Z für Sauerstoff steht,

$R^1$ für Tetrazol steht,
$R^2$ für Wasserstoff steht,
W für Halogen steht, und
X und Y jeweils unabhängig für Wasserstoff stehen,

oder ein pharmazeutisch akzeptables Salz hiervon.

2. Verbindung nach Anspruch 1, worin W für Cl steht.

3. Verbindung nach Anspruch 1 oder 2, die (3S, 4aS, 6S, 8aR)-6-[3-Chlor-2-(1(2)H-tetrazol-5-yl)-phenoxy]-1,2,3,4,4a,5,6,7,8,8a-decahydroisochinolin-3-carbonsäure oder ein pharmazeutisch akzeptables Salz hiervon ist.

4. Verbindung nach Anspruch 3, worin das pharmazeutisch akzeptable Salz das Hydrochloridsalz ist.

5. Verbindung der Formel

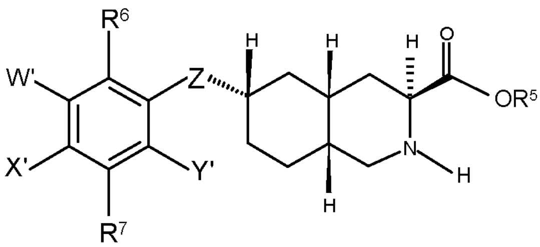

worin

Z für Sauerstoff steht,
$R^5$ für ($C_1$-$C_6$)-Alkyl steht,
$R^6$ für Tetrazol steht,
$R^7$ für Wasserstoff steht,
W für Halogen steht, und
X' und Y' jeweils unabhängig für Wasserstoff stehen,

oder ein pharmazeutisch akzeptables Salz hiervon.

6. Verbindung nach Anspruch 5, worin W' für Cl steht.

7. Verbindung nach Anspruch 5 oder 6, die 2-Ethylbutyl-(3S, 4aS, 6S, 8aR)-6-[3-chlor-2-(1(2)H-tetrazol-5-yl)-phenoxy]-1,2,3,4,4a,5,6,7,8,8a-decahydroisochinolin-3-carboxylat oder ein pharmazeutisch akzeptables Salz hiervon ist.

8. Verbindung nach Anspruch 7, worin das pharmazeutisch akzeptable Salz das Hydrochloridsalz ist.

9. Verbindung nach einem der Ansprüche 1 bis 4 zur Verwendung in der Therapie.

10. Verbindung nach einem der Ansprüche 5 bis 8 zur Verwendung in der Therapie.

11. Verbindung, die 2-Ethylbutyl-(3S, 4aS, 6S, 8aR)-6-[3-chlor-2-(1(2)H-tetrazol-5-yl)-phenoxy]-1,2,3,4,4a,5,6,7,8,8a-decahydroisochinolin-3-carboxylat-para-toluolsulfonat ist, zur Verwendung in der Therapie.

12. Verbindung nach einem der Ansprüche 1 bis 4 zur Verwendung für die Behandlung einer neurologischen Störung oder neurodegenerativen Krankheit.

13. Verbindung nach einem der Ansprüche 5 bis 8 zur Verwendung für die Behandlung einer neurologischen Störung oder neurodegenerativen Krankheit.

14. Verbindung nach Anspruch 12 oder 13, worin die neurologische Störung Schmerz oder Migräne ist.

**15.** Verbindung nach Anspruch 1 oder 5 zur Verwendung für die Behandlung von Schmerz.

**16.** Verbindung nach Anspruch 3 zur Verwendung für die Behandlung von Schmerz.

**17.** Verbindung nach Anspruch 7 zur Verwendung für die Behandlung von Schmerz.

**18.** Verbindung, die 2-Ethylbutyl-(3S, 4aS, 6S, 8aR)-6-[3-chlor-2-(1(2)H-tetrazol-5-yl)-phenoxy]-1,2,3,4,4a,5,6,7,8,8a-decahydroisochinolin-3-carboxylat-para-toluolsulfonat ist, zur Verwendung für die Behandlung von Schmerz.

**19.** Verwendung einer Verbindung nach einem der Ansprüche 1 bis 4 zur Herstellung eines Arzneimittels für die Behandlung einer neurologischen Störung oder neurodegenerativen Krankheit.

**20.** Verwendung einer Verbindung nach einem der Ansprüche 5 bis 8 zur Herstellung eines Arzneimittels für die Behandlung einer neurologischen Störung oder neurodegenerativen Krankheit.

**21.** Verwendung nach Anspruch 19 oder 20, worin die neurologische Störung Schmerz oder Migräne ist.

**22.** Verwendung einer Verbindung nach Anspruch 1 oder 5 zur Herstellung eines Arzneimittels für die Behandlung von Schmerz.

**23.** Verwendung einer Verbindung nach Anspruch 3 zur Herstellung eines Arzneimittels für die Behandlung von Schmerz.

**24.** Verwendung einer Verbindung nach Anspruch 7 zur Herstellung eines Arzneimittels für die Behandlung von Schmerz.

**25.** Verwendung einer Verbindung, die 2-Ethylbutyl-(3S, 4aS, 6S, 8aR)-6-[3-chlor-2-(1 (2)H-tetrazol-5-yl)-phenoxy]-1,2,3,4,4a,5,6,7,8,8a-decahydroisochinolin-3-carboxylat-para-toluolsulfonat ist, zur Herstellung eines Arzneimittels für die Behandlung von Schmerz.

**26.** Pharmazeutische Zusammensetzung, umfassend eine wirksame Menge der Verbindung nach einem der Ansprüche 1 bis 4 in Kombination mit einem pharmazeutisch akzeptablen Träger, Verdünnungsmittel oder Exzipient.

**27.** Pharmazeutische Zusammensetzung, umfassend eine wirksame Menge der Verbindung nach einem der Ansprüche 5 bis 8 in Kombination mit einem pharmazeutisch akzeptablen Träger, Verdünnungsmittel oder Exzipient.

**28.** Pharmazeutische Zusammensetzung, umfassend eine wirksame Menge der Verbindung, die 2-Ethylbutyl-(3S, 4aS, 6S, 8aR)-6-[3-chlor-2-(1(2)H-tetrazol-5-yl)-phenoxy]-1,2,3,4,4a,5,6,7,8,8a-decahydroisochinolin-3-carboxylat-para-toluolsulfonat ist, in Kombination mit einem pharmazeutisch akzeptablen Träger, Verdünnungsmittel oder Exzipient.

**29.** Verfahren zur Herstellung einer Verbindung der Formel,

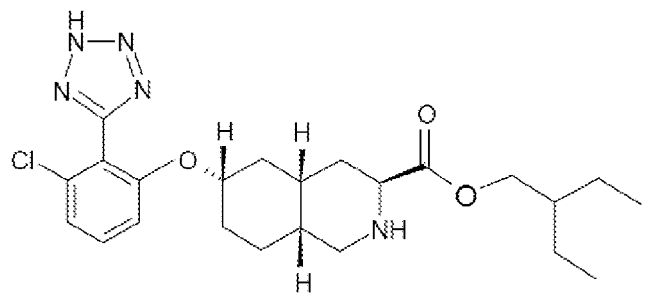

umfassend eine Kombination einer Verbindung der Struktur

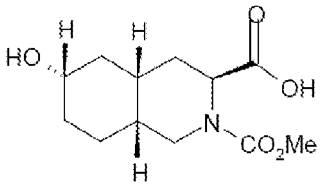

mit einer geeigneten Base in einem geeigneten Lösemittel, eine anschließende Zugabe einer Verbindung der Struktur

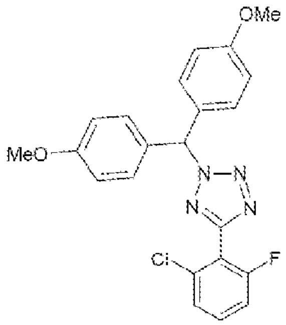

eine anschließende Veresterung zu einer Verbindung der Struktur

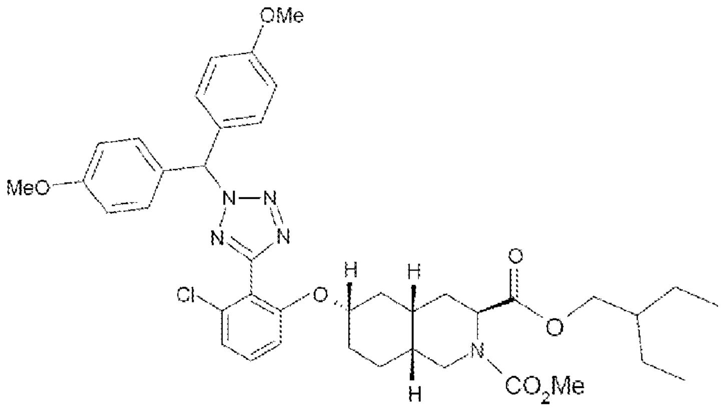

eine anschließende Abspaltung der Stickstoffschutzgruppe,
und eine Ausfällung mit einer geeigneten Säure.

**30.** Verfahren zur Synthese einer Verbindung der Formel

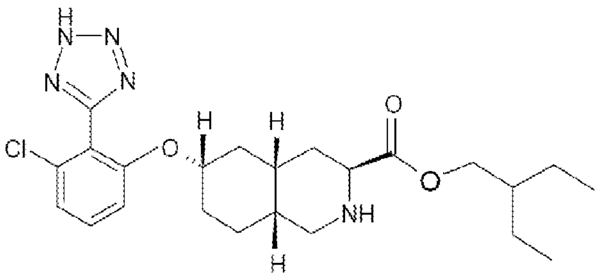

umfassend eine Kombination einer Verbindung der Struktur

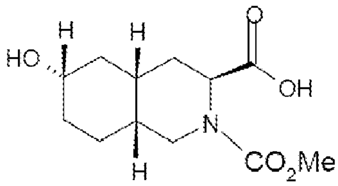

mit einer geeigneten Base in einem geeigneten Lösemittel, einen anschließenden Zusatz einer Verbindung der Struktur

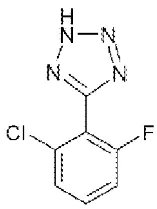

eine anschließende Abspaltung der Stickstoffschutzgruppe, eine Ausfällung mit einer geeigneten Säure und eine Kristallisation des Hydratsalzes der Struktur

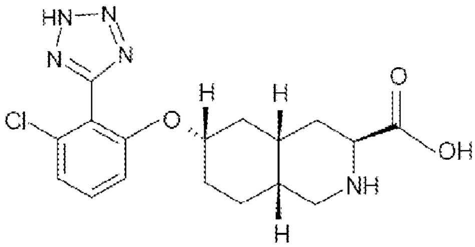

gefolgt von einer Behandlung mit einem geeigneten Alkohol in Gegenwert einer geeigneten Säure zur Bewirkung der einstufigen Veresterung und einer Kristallisation der Verbindung der Formel

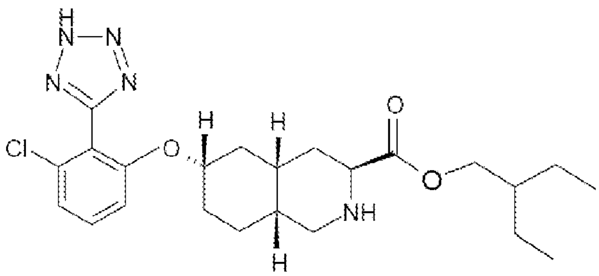

**31.** Verfahren zur Herstellung von 2-Ethylbutyl-(3S, 4aS, 6S, 8aR)-6-[3-chlor-2-(1(2)H-tetrazol-5-yl)-phenoxy]-1,2,3,4,4a,5,6,7,8,8a-decahydroisochinolin-3-carboxylat-para-toluolsulfonat, umfassend eine Kombination einer Verbindung der Struktur

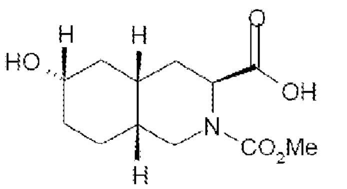

mit einer geeigneten Base in einem geeigneten Lösemittel unter anschließender Zugabe einer Verbindung der Struktur

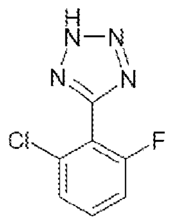

gefolgt von einer Spaltung der Stickstoffschutzgruppe, einer Ausfällung mit HCl und einer Kristallisation des Monohydrathydrochloridsalzes der Verbindung der Struktur

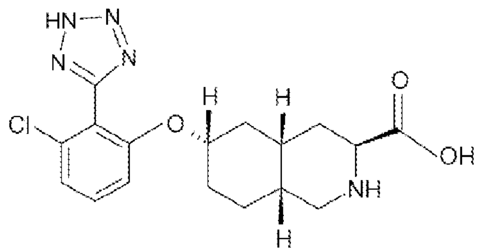

gefolgt von einer Behandlung mit einem 2-Ethyl-1-butanol in Gegenwart von para-Toluolsulfonsäure zwecks Bewirkung der einstufigen Veresterung und einer Kristallisation von 2-Ethylbutyl-(3S, 4aS, 6S, 8aR)-6-[3-chlor-2-(1(2) H-tetrazol-5-yl)-phenoxy]-1,2,3,4,4a,5,6,7,8,8a-decahydroisochinolin-3-carboxylat-para-toluolsulfonat.

## Revendications

**1.** Composé de formule

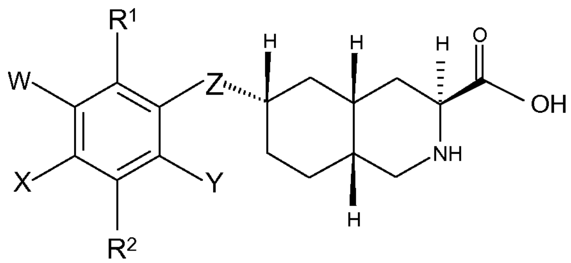

dans laquelle

Z représente un atome d'oxygène ;
$R^1$ représente un groupe tétrazole ;
$R^2$ représente un atome d'hydrogène ;
W représente un groupe halogéno ; et
X, et Y représentent chacun indépendamment un atome d'hydrogène ;
ou un sel pharmaceutiquement acceptable de celui-ci.

**2.** Composé selon la revendication 1, dans lequel W est Cl.

**3.** Composé selon la revendication 1 ou 2, qui est l'acide (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylique ou un sel pharmaceutiquement acceptable de

celui-ci.

4. Composé selon la revendication 3, dans lequel le sel pharmaceutiquement acceptable est le sel chlorhydrate.

5. Composé de formule :

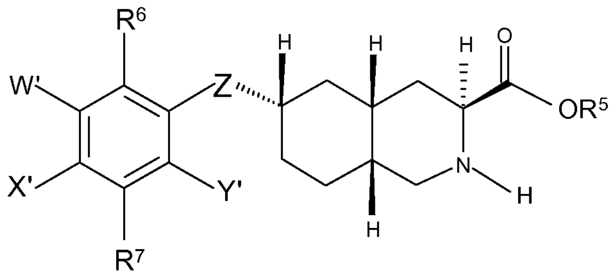

dans laquelle,

Z représente un atome d'oxygène ;
$R^5$ représente un groupe alkyle en ($C_1$-$C_6$) ;
$R^6$ représente un groupe tétrazole ;
$R^7$ représente un atome d'hydrogène ;
W' représente un groupe halogéno ; et
X' et Y' représentent chacun indépendamment un atome d'hydrogène ;
ou un sel pharmaceutiquement acceptable de celui-ci.

6. Composé selon la revendication 5, dans lequel W' représente Cl.

7. Composé selon la revendication 5 ou 6 qui est le (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylate de 2-éthyl-butyle ou un sel pharmaceutiquement acceptable de celui-ci.

8. Composé selon la revendication 7, dans lequel le sel pharmaceutiquement acceptable est le sel chlorhydrate.

9. Composé selon l'une quelconque des revendications 1 à 4 pour l'utilisation en thérapie.

10. Composé selon l'une quelconque des revendications 5 à 8 pour l'utilisation en thérapie.

11. Composé qui est le (3*S*, 4a*S*, 6*S*, 8a*R*) 6-[3-chloro-2-(1(2)*H*-tétrazol-5-yl)-phénoxy]-1,2,3,4,4a,5,6,7,8,8a-décahydroisoquinoléine-3-carboxylate para-toluènesulfonate de 2-éthyl-butyle pour l'utilisation en thérapie.

12. Composé selon l'une quelconque des revendications 1 à 4 pour l'utilisation dans le traitement d'un trouble neurologique ou d'une maladie neurodégénérative.

13. Composé selon l'une quelconque des revendications 5 à 8 pour l'utilisation dans le traitement d'un trouble neurologique ou d'une maladie neurodégénérative.

14. Composé selon la revendication 12 ou la revendication 13, dans lequel le trouble neurologique est la douleur ou la migraine.

15. Composé selon la revendication 1 ou la revendication 5 pour l'utilisation dans le traitement de la douleur.

16. Composé selon la revendication 3 pour l'utilisation dans le traitement de la douleur.

17. Composé selon la revendication 7 pour l'utilisation dans le traitement de la douleur.

18. Composé qui est le (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylate para-toluènesulfonate de 2-éthyl-butyle pour l'utilisation dans le traitement de la douleur.

**19.** Utilisation d'un composé selon l'une quelconque des revendications 1 à 4, pour la fabrication d'un médicament pour le traitement d'un trouble neurologique ou d'une maladie neurodégénérative.

**20.** Utilisation d'un composé selon l'une quelconque des revendications 5 à 8, pour la fabrication d'un médicament pour le traitement d'un trouble neurologique ou d'une maladie neurodégénérative.

**21.** Utilisation selon la revendication 19 ou la revendication 20, dans laquelle le trouble neurologique est la douleur ou la migraine.

**22.** Utilisation d'un composé selon la revendication 1 ou la revendication 5, pour la fabrication d'un médicament pour le traitement de la douleur.

**23.** Utilisation d'un composé selon la revendication 3, pour la fabrication d'un médicament pour le traitement de la douleur.

**24.** Utilisation d'un composé selon la revendication 7, pour la fabrication d'un médicament pour le traitement de la douleur.

**25.** Utilisation d'un composé qui est le (3*S*, 4a*S*, 6*S*, 8a*R*)6-[3-chloro-2-(1 (2)H-tétrazol-5-yl)-phénoxy]-1,2,3,4,4a, 5,6,7,8,8a-décahydroisoquinoléine-3-carboxylate para-toluènesulfonate de 2-éthyl-butyle pour la fabrication d'un médicament pour le traitement de la douleur.

**26.** Composition pharmaceutique comprenant une quantité efficace du composé selon l'une quelconque des revendications 1 à 4, en combinaison avec un support, un diluant, ou un excipient pharmaceutiquement acceptable.

**27.** Composition pharmaceutique comprenant une quantité efficace du composé selon l'une quelconque des revendications 5 à 8, en combinaison avec un support, un diluant, ou un excipient pharmaceutiquement acceptable.

**28.** Composition pharmaceutique comprenant une quantité efficace du composé qui est le (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylate para-toluènesulfonate de 2-éthyl-butyle, en combinaison avec un support, un diluant, ou un excipient pharmaceutiquement acceptable.

**29.** Procédé de préparation d'un composé de formule :

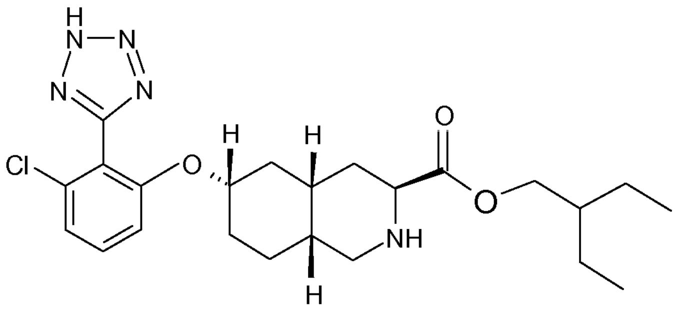

comprenant la combinaison d'un composé de structure :

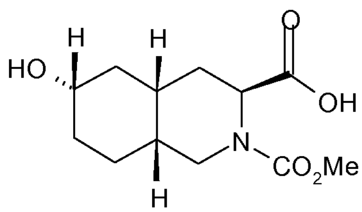

avec une base appropriée dans un solvant approprié, suivie de l'addition d'un composé de structure :

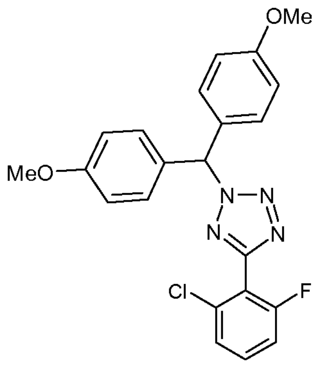

suivie de l'estérification en un composé de structure :

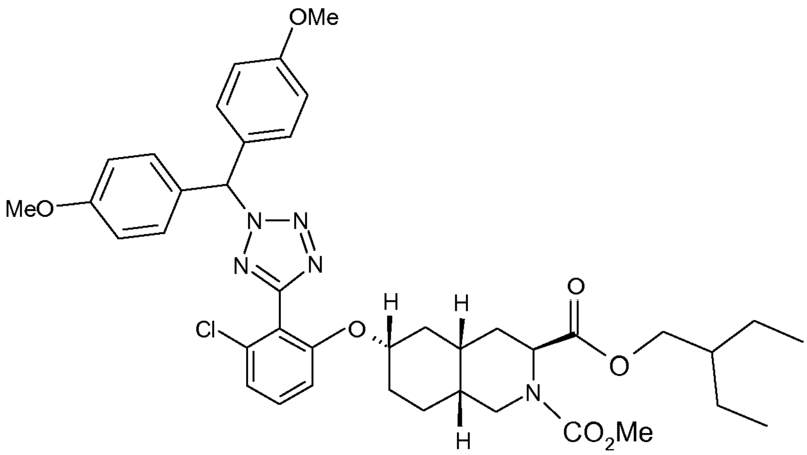

suivie de l'élimination des groupes protecteurs de l'atome d'azote, et de la précipitation avec un acide approprié.

**30.** Procédé de synthèse d'un composé de formule :

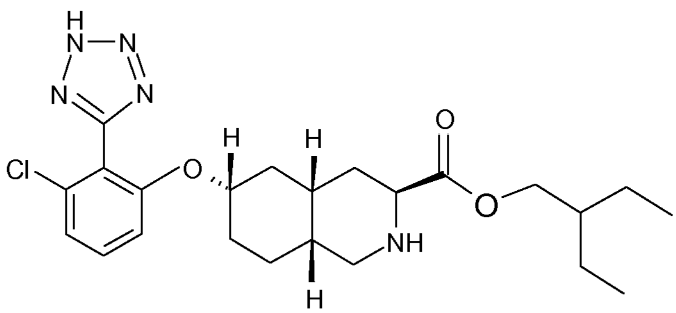

comprenant la combinaison d'un composé de structure :

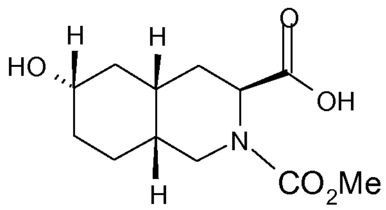

avec une base appropriée dans un solvant approprié, suivie de l'addition d'un composé de structure :

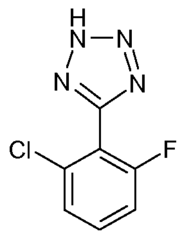

suivie de la déprotection du groupe azote, de la précipitation avec un acide approprié, et de la cristallisation du sel hydrate de structure :

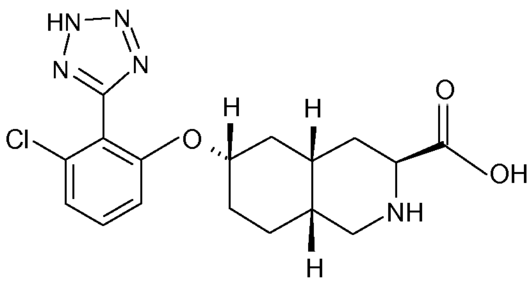

suivie du traitement avec un alcool approprié en la présence d'un acide approprié pour effectuer l'estérification en une étape et la cristallisation du composé de formule :

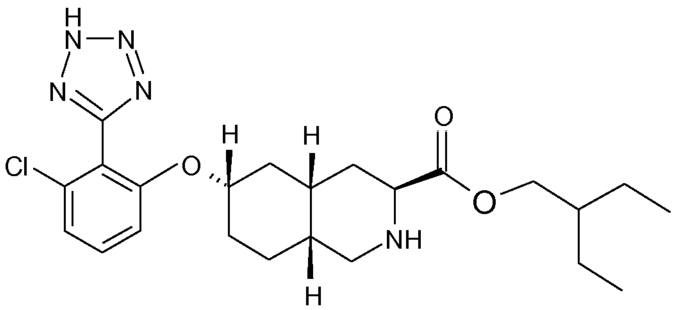

**31.** Procédé de préparation du (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylate para-toluènesulfonate de 2-éthyl-butyle, comprenant la combinaison d'un composé de structure :

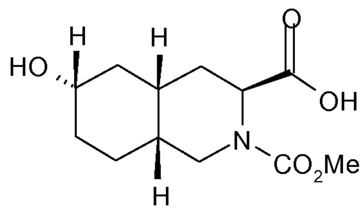

avec une base appropriée dans un solvant approprié, suivie de l'addition d'un composé de structure :

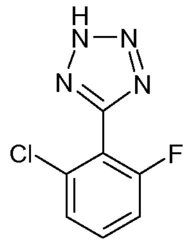

suivie de la déprotection du groupe protecteur de l'atome d'azote, de la précipitation avec HCl, et de la cristallisation du sel chlorhydrate monohydraté du composé de structure :

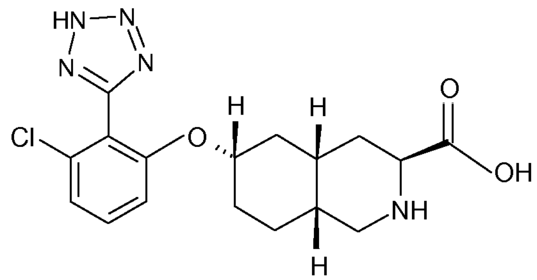

.

suivie du traitement avec du 2-éthyl-1-butanol en la présence d'acide para-toluènesulfonique pour effectuer l'estérification en une étape et la cristallisation du (3*S*, 4a*S*, 6*S*, 8a*R*)6-[3-chloro-2-(1(2)*H*-tétrazol-5-yl)-phénoxy]-1,2,3,4,4a,5,6,7,8,8a-décahydroisoquinoléine-3-carboxylate para-toluènesulfonate de 2-éthyl-butyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5446051 A
- US 5670516 A
- WO 9845720 A
- EP 590789 A1
- WO 9845270 A
- US 5356902 A
- US 4902695 A
- WO 00028980 A


### Non-patent literature cited in the description


- **WATKINS ; EVANS.** *Ann. Rev. Pharmacol. Toxicol.,* 1981, vol. 21, 165
- **MONAGHAN ; BRIDGES ; COTMAN.** *Ann. Rev. Pharmacol. Toxicol.,* 1989, vol. 29, 365
- **WATKINS ; KROGSGAARD-LARSEN ; HONORE.** *Trans. Pharm. Sci.,* 1990, vol. 11, 25
- **BLEAKMAN et al.** *Molecular Pharmacology,* 1996, vol. 49 (4), 581
- **SCHOEPP ; CONN.** *Trends in Pharmacol. Sci.,* 1993, vol. 14, 13
- **SCHOEPP ; BOCKAERT ; SLADECZEK.** *Trends in Pharmacol. Sci.,* 1990, vol. 11, 508
- **MCDONALD ; JOHNSON.** *Brain Research Reviews,* 1990, vol. 15, 41
- **MOSKOWITZ.** *Cephalalgia,* 1992, vol. 12, 5-7
- **MACINTYRE, P.D. et al.** *British Journal of Clinical Pharmacology,* 1992, vol. 34, 541-546
- **CHESTER, A.H. et al.** *Cardiovascular Research,* 1990, vol. 24, 932-937
- **CONNER, H.E. et al.** *European Journal of Pharmacology,* 1990, vol. 161, 91-94
- **SHEARDOWN et al.** *Science,* 1900, vol. 247, 571
- **BUCHAN et al.** *Neuroreport,* 1991, vol. 2, 473
- **LEPEILLET et al.** *Brain Research,* 1992, vol. 571, 115
- **LAPEILLET et al.** *Brain Research,* 1992, vol. 571, 115
- Enantiomers, Racemates, and Resolutions. **J. JACQUES et al.** Enantiomers, Racemates, and Resolutions. John Wiley and Sons, Inc, 1981
- *Nomenclature of Organic Compounds: Principles and Practice,* 1974, 103-120
- **ELIEL ; WILEN.** Stereochemistry of Organic Compounds. John Wiley & Sons, Inc, 1994
- **COLLET ; WILEN.** Enantiomers, Racemates, and Resolutions. John Wiley & Sons, Inc, 1981
- **GREENE.** Protective Groups In Organic Synthesis. John Wiley & Sons, 1981
- **KORCZAK et al.** *Recept. Channels,* 1994, vol. 3, 41-49
- **BLEAKMAN et al.** *Mol. Pharmacol.,* 1996, vol. 49, 581-585
- **FLETCHER et al.** *Recept. Channels,* 1995, vol. 3, 21-31
- **HOO et al.** *Recept. Channels,* vol. 2, 327-338
- **FLETCHER ; LODGE.** *Pharmacol. Ther.,* 1996, vol. 70, 65-89
- **SHIBATA et al.** *Pain,* 1989, vol. 38, 347-352
- **WHEELER-ACETO et al.** *Pain,* 1990, vol. 40, 229-238
- **CODERRE et al.** *Eur. J. Neurosci.,* 1993, vol. 6, 1328-1334
- **ABBOTT et al.** *Pain,* 1995, vol. 60, 91-102
- **HAUBRICH et al.** Pharmacology of pravadoline: a new analgesic agent. *The Journal of Pharmacology and Experimental Therapeutics,* 1990, vol. 255, 511-522
- **HARGREAVES et al.** *Pain,* 1988, vol. 32, 77-88
- **HARGREAVES et al.** *Plain,* 1988, vol. 32, 77-88
- **R.E. KIRK.** Experimental Design: Procedures for the behavioral sciences. 1982